# EUROPEAN PATENT SPECIFICATION

(11) **EP 3 987 062 B1**
(45) Date of publication and mention of the grant of the patent: **21.05.2025**
(21) Application number: 20733449.1
(22) Date of filing: 22.06.2020
(51) Int. Cl.: C12Q 1/6886

(54) **DETECTION OF HYPERMETHYLATED GENES FOR DIAGNOSING GASTRIC CANCER**
DETEKTION VON HYPERMETHYLIERTEN GENEN ZUR DIAGNOSE VON MAGENKREBS
DÉTECTION DE GÈNES HYPERMÉTHYLÉS POUR LE DIAGNOSTIC DU CANCER DU PANCRÉAS

(30) Priority: 21.06.2019 EP 19305811
(43) Date of publication of application: 27.04.2022
(73) Proprietor: Université Paris Cité, 75006 Paris (FR); CENTRE NATIONAL DE LA RECHERCHE SCIENTIFIQUE (CNRS), 75016 Paris (FR); Assistance Publique - Hôpitaux de Paris, 75004 Paris (FR); INSTITUT NATIONAL DE LA SANTE ET DE LA RECHERCHE MEDICALE (INSERM), 75013 Paris (FR)
(72) Inventor: WANG-RENAULT, Shufang, 92340 BOURG LA REINE (FR); TALY, Valérie, 92340 BOURG LA REINE (FR); LAURENT-PUIG, Pierre, 92190 MEUDON (FR); ZAANAN, Aziz, 92100 BOULOGNE BILLANCOURT (FR)
(74) Representative: Cabinet Becker et Associés
(86) International application number: PCT/EP2020/067370
(87) International publication number: WO 2020/254693

(56) References cited:
- WO-A1-2019/068082
- WO-A2-2009/034481
- US-A1- 2015 038 352
- US-A1- 2017 058 356
- QU YIPING ET AL: "Gene methylation in gastric cancer", CLINICA CHIMICA ACTA, ELSEVIER BV, AMSTERDAM, NL, vol. 424, 10 May 2013 (2013-05-10), pages 53 - 65, XP028708538, ISSN: 0009-8981, DOI: 10.1016/J.CCA.2013.05.002

## Description

### Summary of the invention

The present invention relates to a method for diagnosing or identifying a gastric cancer in a subject, through the detection of the abnormal hypermethylation levels of specific genes in a biological sample of said subject. The inventors indeed identified three DNA methylation biomarkers that, alone or in combination, can help diagnosing or following-up gastric cancer patients. They propose to measure the DNA hypermethylation of said genes by dPCR, in a blood sample of the subject. The methylated genes are preferably chosen from the group consisting of: *ZNF790-AS1, MSC-AS1* and *KCNA3.* The present invention also relates to kits and other tools for diagnosing gastric cancers. The invention is defined by the claims. Any subject-matter falling outside the scope of the claims is provided for information purposes only.

### Background prior art

Stomach cancer, also known as gastric cancer, is a cancer developing from the lining of the stomach. Early symptoms may include heartburn, upper abdominal pain, nausea and loss of appetite, dysphagia. Later signs and symptoms may include weight loss, anorexia, vomiting, hematemesis, persistent ulcer, difficulty swallowing and blood in the stool among others. The cancer may spread from the stomach to other parts of the body, particularly the liver, lungs, bones, lining of the abdomen and lymph nodes. Most cases of stomach cancers are gastric carcinomas. Lymphomas and mesenchymal tumors may also develop in the stomach. Most of the time, stomach cancer develops in stages over years. The prognosis of this disease is linked to the tumor stage at diagnosis. More the disease is advanced and more the prognosis is poor. In case of metastasis disease, the 5-year survival rate is less than 5%, highlighting the importance of early diagnosis for this disease.

The most common cause is infection by the bacterium *Helicobacter pylori,* which accounts for more than 60% of cases. Certain types of *H. pylori* have greater risks than others. Smoking, dietary factors such as pickled vegetables and obesity are other risk factors. About 10% of cases run in families, and between 1% and 3% of cases are due to genetic syndromes inherited from a person's parents such as hereditary diffuse gastric cancer. Globally, stomach cancer is the fifth leading cause of cancer and the third leading cause of death from cancer, making up 7% of cases and 9% of deaths. In 2012, it newly occurred in 950,000 people and caused 723,000 deaths.

Diagnosis of gastric cancer is usually done by biopsy during endoscopy. This is followed by medical imaging to determine if the disease has spread to other parts of the body. There is no approved blood-derived biomarker associated specifically with gastric cancer. Japan and South Korea, two countries that have high rates of the disease, already screen for stomach cancer by gastroscopy, ultrasound tests, and the like. However, these methods require expensive medical equipment that generate considerable costs. Moreover, some patients may be reluctant to undergo gastroscopy.

In this context, there is still a need to identify a sensitive and specific biomarker of gastric cancer, that can be used on blood samples by easy means.

The identification of candidate cancer biomarkers has exploded since the introduction of genome-wide sequencing of diseased tissue DNA using next-generation sequencing (NGS). However, such identification is useful for the early detection of cancer only if it can be done in the periphery, noninvasively. Unlike a mutation, which is a specific change in the DNA sequence and hence can be recognized unambiguously, DNA methylation often presents in multiple CpG sites and has a spectrum of quantitative differences between tumor and normal tissues.

Methylation of the promoter and the first exon of tumor suppressor genes resulting in downregulation of gene expression has been shown to play a crucial role in tumorigenesis. Aberrant DNA methylation often occurs in the early stage in carcinogenesis, thus providing attractive potential markers for the early detection of cancer. For developing biomarkers for cancer screening and early detection, the increased methylation (hypermethylation) provides a positive readout with clear target regions for sensitive and specific assay development, and thus is advantageous over global hypomethylation for a clinical test [Jain, Wojdacz & Su, 2013].

Despite these attractive promises, challenges exist for translating methylated DNA marker candidates into useful clinical diagnosis markers.

Several reviews have discussed the obstacles limiting the routine use of DNA methylation biomarkers in cancer therapy [Mikeska T, 2012; Jain, Wojdacz & Su, 2013; Delpu Y et al, 2013].

These reviews highlight that analytical sensitivity is a critical parameter for diagnostic applicability of methylation screening technology and that a good analytical sensitivity can be achieved only by combining several markers or by complementing pre-existing screening tests, because a single-marker approach is unlikely to have a sufficient sensitivity (>90%) for screening, except in extremely well-defined high-risk groups where the marker is linked with the etiology of cancer. Also WO2019/068082 A1 (UNIV ARIZONA [US]; VRBA LUKAS [US]; FUTSCHER BERNARD W [US]) describes methylation marker sets for cancer detection. Furthermore WO2009/034481 A2 (KOREA RES INST OF BIOSCIENCE [KR]; KIM YONG SUNG [KR]) and QU YIPING et al. (CLINICA CHIMICA ACTA, vol. 424, 10 May 2013 pages 53-65) describe methylation markers for the detection of gastric cancer. US2017/058356 A1 (AHLQUIST DAVID A [US] ET AL) describes methylation marker panels for the detection of gastric cancer, which include KCNA3.

Another critical factor for a screening or a diagnosis test is high specificity. Low specificity results in high numbers of false-positive results, which may lead to mental stress for patients and to unnecessary medical procedures, undermining the credibility and acceptance of the test in the population. The local circumstances in a methylation event require an assay to be quantitative enough to distinguish the truly cancer-related hypermethylation event from baseline methylation. It has also been reported that DNA methylation biomarkers are often not specific of one cancer but mostly conserved among tumor types. Thus, it seems challenging to propose a single DNA methylation alteration as a biomarker for a certain type of cancer, what is nevertheless ideal in order to treat the patient with an appropriate treatment as early as possible. Combination of several biomarkers has often been proposed to compensate for this lack of specificity, yet it also lowers the sensitivity of the test.

Altogether, the above-mentioned reviews show that, although proof of principle is already available for the great potential of methylation biomarkers in clinical use, the field faces many challenges for identifying ideal biomarkers exhibiting both a high sensitivity and a high specificity.

Moreover, none of these reviews discloses any study highlighting a methylation biomarker for diagnosing gastric cancer. This suggests that it is quite difficult to identify a sensitive and specific methylation biomarker of gastric cancer.

In this scientific context, the present inventors however observed, as disclosed in the experimental part below, that it is possible to use three different specific methylation biomarkers, either alone or in combination, for diagnosing, monitoring or following-up gastric cancer with an excellent sensitivity and specificity. These findings contrast with all the drawbacks suggested by the specialists in the field in the reviews cited above.

### Detailed description of the invention

In the field of cancer biomarkers, the most widely studied epigenetic modification is cytosine methylation. DNA methylation in the human genome occurs mostly at the cytosine residues in a CpG dinucleotide at the carbon-5 position, resulting in 5-methylcytosine. CpG dinucleotides are rare in the human genome (~1%). CpG dinucleotides are often found to be in clusters of more than 200 bases with more than 50% of G+C content and a ratio of CpG frequencies of at least 0.6, which are known as CpG islands. Approximately 60% of the human gene promoters are associated with CpG islands. The CpG islands within the promoter regions are usually unmethylated in normal cells, except for some involved in tissue differentiation. In general, CpG island methylation is associated with transcriptional silencing. In cancer both global hypomethylation (decrease of overall DNA methylation) and localized hypermethylation, such as methylation of the promoter and the first exon of tumor suppressor genes, have been observed. DNA hypomethylation occurs at many genomic sequences, such as repetitive elements, retrotransposons, introns and similar elements, resulting in genomic instability, and can account for the activation of some proto-oncogenes and lead to loss of imprinting, as in the case of the *IGF2* gene (encoding IGF-2) in Wilms' tumor [Jain, Wojdacz & Su, 2013].

To our knowledge, no study has ever revealed any DNA methylation biomarker for diagnosing gastric cancer specifically.

Yet, the present inventors have observed that specific regions within the promoter or introns of three specific genes (namely *MSC-AS1, ZNF790-AS1 & KCNA3*) are abnormally hypermethylated in gastric cancer tumor samples specifically. Interestingly, two of these three genes (namely *MSC-AS1 & ZNF790-AS1)* were never found associated with cancer diagnostic or progression. The third gene (*KCNA3*) had never been associated to gastric cancer.

The *"MSC-AS1* gene" of the invention designates in fact a non-coding RNA called "MSC antisense RNA 1". Its DNA sequence is located on chromosome 8 (71 71843123 - 72 056312), more precisely between 8q19.3 and 8q21.11. It contains 5 exons. Its DNA sequence is referred to as NC_000008.11. It is transcribed into two RNA variants indexed as NR_033651.1 and NR_033652.1 respectively. The present inventors have identified the DNA region comprised between 72755486 and 72756187 (SEQ ID NO:4), more preferably 72755783 and 72756187 (SEQ ID NO:1) to be significantly hypermethylated specifically in gastric tumor samples, by contrast with healthy samples. They therefore propose to use this gene in a non-invasive, sensitive and reliable method for diagnosing or identifying gastric cancer in a subject, or in kits useful for such purposes.

The *"ZNF790-AS1* gene" of the invention designates a non-coding RNA, called "ZNF790 antisense RNA 1". It has also been referred to as "CTD-2162K18.5". Its DNA sequence is located on chromosome 19 (36 797550 - 36 828111), more precisely on 19q13.12. It contains 6 exons. Its DNA sequence is referred to as NC_0000019.10. It is transcribed into two RNA variants indexed as NR_040027.1 and NR_040028.1 respectively. Hypermethylation of its promoter region has never been reported. The present inventors have identified the DNA region comprised between 37288170 and 37288811 (SEQ ID NO:5), more preferably 37288170 and 37288450 (SEQ ID NO:2) to be significantly hypermethylated specifically in gastric tumor samples, by contrast with healthy samples. They therefore propose to use this gene in a non-invasive, sensitive and reliable method for diagnosing or identifying gastric cancer in a subject, or in kits useful for such purposes.

The *"KCNA3* gene" of the invention designates the gene encoding the potassium voltage gated channel subfamily A member 3 protein, also known as "HPCN3", "HGK5", "HLK3", "HUKIII", "KV1.3", "MK3", "PCN3". It is expressed in T and B lymphocytes and is involved in autoimmune diseases, obesity, and Alzheimer disease. Its DNA sequence is located on chromosome 1 (110 653560 - 110 675033), more precisely on 1p13.3. It contains 4 exons. Its DNA sequence is referred to as NC_000001.11. It is transcribed into the mRNA indexed as NM_002232.4 and transcribed into the protein indexed as NP_002223.3. The present inventors have identified the DNA region comprised between 111217406 and 111218015 (SEQ ID NO:6), more preferably 111217406 and 111217815 (SEQ ID NO:3), encompassing partially the promoter and the first exon of the gene, to be significantly hypermethylated specifically in gastric tumor samples, by contrast with healthy samples or reference sample of said patients. They therefore propose to use this gene in a non-invasive, sensitive and reliable method for diagnosing or identifying gastric cancer or performing a follow up of gastric cancer in a subject.

The inventors have shown that hypermethylation of the promoter or other regions of any of these genes can be used as a sensitive and specific biomarker of gastric cancer in patients suffering thereof, even at early stage. The *MSC-AS1* gene, *ZNF790-AS1* gene, and *KCNA3* gene are hereafter referred to as the "biomarkers of the invention". They can be used individually, or in combination two by two, or all the three together.

### Methods of the invention

In a first aspect, the present invention therefore relates to an *in vitro* method for diagnosing or identifying gastric cancer in a subject, said method comprising determining in a biological sample of said subject the level or amount of methylation of at least one gene, preferably two genes selected from the group consisting of: the *ZNF790-AS1* gene, the *MSC-AS1* gene, and the *KCNA3* gene that are described above. In a particularly preferred embodiment, said method comprises determining the level or amount of methylation of the three genes *ZNF790-AS1, MSC-AS1,* and *KCNA3* described above.

In a preferred embodiment, said method comprises determining the level or amount of methylation in the nucleotide region of SEQ ID NO:4 in the *MSC-AS1* gene or in the nucleotide region of SEQ ID NO:5 in the *ZNF790-AS1* gene or in the nucleotide region of SEQ ID NO:6 in the *KCNA3* gene.

In a more preferred embodiment, said method comprises determining the level or amount of methylation in the nucleotide region of SEQ ID NO:1 in the *MSC-AS1* gene or in the nucleotide region of SEQ ID NO:2 in the *ZNF790-AS1* gene or in the nucleotide region of SEQ ID NO:3 in the *KCNA3* gene.

In a more preferred embodiment, said method comprises determining simultaneously or sequentially the level or amount of methylation of the *MSC-AS1* gene, the *ZNF790-AS1* gene and the *KCNA3* gene in a biological sample of said subject.

As used herein, the term "subject" refers to a mammal, preferably a human. Preferably, it refers to a human patient that may be healthy (without any symptoms of gastric cancer), or that is thought to develop or is suspected of suffering from cancer, preferably gastric cancer. Said subject for example presents at least one of the following symptoms: heartburn, upper abdominal pain, nausea, loss of appetite, weight loss, vomiting, difficulty swallowing and blood in the stool. Said subject may also have suffered from a gastric cancer in the past, has been treated, and is monitored for potential disease recurrence. Said subject may also seem to be healthy but is predisposed to develop a gastric cancer, for example genetic, family history such as a member of his family is suffering or has suffered from the same disease or has a genetic predisposition such as Lynch syndrome or germinal CDH1 mutation.

As used herein, the expression "biological sample" refers to solid tissues such as, for example, a gastrointestinal biopsy or to fluids, body effluents and excretions such as for example, sputum, induced sputum, blood, serum, plasma, urine, feces. Preferably, said biological sample is a fluid sample and most preferably a blood or plasma sample.

In the method of the invention, if the said gene is hypermethylated as compared with a reference sample, then said subject is diagnosed or identified as suffering from a gastric cancer. In other words, a high level or amount of methylation is regarded as an indicator of gastric cancer, of invasive high-grade gastric cancer or of relapse of gastric cancer. In another aspect, said methylation can be compared with the methylation of a previous sample of said subject, and then said subject is diagnosed or not as suffering from a gastric cancer.

A number of techniques has been proposed to detect DNA methylation. The review of Delpu et al (2013) discloses some of them:
Methylation specific-PCR (MS-PCR) is a method in which two sets of PCR primers are specifically designed to amplify methylated and unmethylated DNA regions of interest. The detection of PCR products is originally performed by gel electrophoresis. This technique has been replaced by Quantitative MS-PCR (qMS-PCR), in which PCR amplification is monitored in real time by the incorporation of fluorescent molecules. This improvement allows for precise quantification of the DNA methylation levels of numerous specific regions and avoids the long electrophoresis step. Quantitative multiplex MS-PCR (QM-MS-PCR) and one step MS-PCR (OS-MS-PCR) are also available to co-amplify specific genes in tissues from different origins or to determine DNA methylation levels of a specific region without the DNA extraction procedure. qMS-PCR techniques are simple, rapid, inexpensive, highly sensitive and easily standardized. They are currently one of the most commonly used techniques for cancer diagnosis in clinical use. Methylation-sensitive high-resolution melting (MS-HRM) is based on the fact that the nucleotide sequence of PCR products of bisulfite-treated DNA will differ depending on the methylation status of the DNA region of interest. The methylation level is determined by comparing the melting dissociation curves to standard PCR products of the same region containing known methylated CpG sites. COBRA, for combined bisulfite restriction analysis, uses the ability of bisulfite conversion to create new restriction enzyme sites or to maintain consensus sites of MSRE. After amplification, PCR products are digested with appropriate MSRE. The proportion of digested PCR products is compared to undigested PCR products by poly-acrylamide gel electrophoresis and image quantification software. This technique is reliably applied to DNA obtained from formalin-fixed paraffin embedded (FFPE) tissue sample. Moreover, this approach allows for the assessment of the DNA methylation of a large number of biological samples. More recently, high throughput approaches have been developed. For instance, Methyl Light is a high throughput quantitative methylation assay that uses fluorescent-based real time PCR (TaqMan^{®}, Applied Biosystems, Forster City, CA, USA) in combination to bisulfite treatment. Also combined with bisulfite treatment pyrosequencing is a quantitative DNA sequencing method in which light is emitted as a result of an enzymatic reaction representing each time a nucleotide is incorporated into the growing DNA chain. These quantitative techniques detect low amounts of methylated DNA in heterogeneous DNA preparation. Easily standardized, rapid and inexpensive, these techniques are increasingly used for clinical purpose.

More recently, next-generation sequencing (NGS) technologies significantly increased the resolution level of DNA methylation profiles. NGS can also be adapted to immuno-precipitated DNA fragment (Methyl DNA Immuno-precipitation sequencing also called MeDIP seq). Ultimately NGS permits the sequencing of the entire genome after bisulfite conversion. Beside these NGS approaches, high throughput single nucleotide polymorphism (SNP) genotyping systems are suitable for DNA methylation analysis from bisulfite-converted genomic DNA. Further, it is possible to use other methods well known to the skilled person, such as methods which directly analyze the unmodified DNA (for example nanopore sequencing), methods using specific restriction enzymes, methylated sequence enrichment methods (e.g. EpiMark methylated Enrichment kit, New England), immunoprecipitation methods.

All these methods (qMS-PCR, MS-HRM, COBRA, MSRE, Methyl Light, NGS, SNP genotyping, pyrosequencing, microarray, ICE-cold PCR, nanopore etc.) can be used for determining the methylation of the markers of the invention.

The method of the invention requires to detect the "level of methylation", "methylation level" or the "amount of methylation" in CpG sites, depending on the detection technology which is used. According to the present invention, the terms "level of methylation" or "amount of methylation" refer to the determination of a quantitative measure. Thus, the terms "level of methylation" or "amount of methylation" can be used interchangeably.

According to the present invention, the "reference sample" which is used to detect an "hypermethylation" for carrying out a diagnostic of gastric cancer or for following the evolution of gastric cancer is a biological sample from a subject that does not suffer from gastric cancer or biological sample from a subject who has been previously diagnosed as suffering from gastric cancer such as a normal or healthy cell or tissue or body fluid, or a data set produced using information from a normal or healthy cell or tissue or body fluid. For example, said reference sample can be genomic DNA extracted from total blood or circulated DNA extracted from healthy subjects where said samples are the same as the one tested for the patients (for example plasma). In a particular embodiment of the invention, the reference sample can be different depending the type of biological sample used. For example, the tissue sample may be either a sample identified as non tumorogenous in the tissue surrounding the tumor (normal adjacent tissue) or a tissue from a healthy part of the same organ (not adjacent).

According to another example, the reference blood sample for diagnosis or screening can be a sample from a subject that does not suffer from cancer (including gastric cancer) such as a normal or healthy cell or tissue or body fluid, or a sample from a subject who has been previously diagnosed as suffering from gastric cancer such as a normal or healthy cell or tissue, or a data set produced using information from a normal or healthy cell or tissue or body fluid. Further, the blood sample for follow-up of cancer patients can be a biological sample from a subject that does not suffer from cancer (including gastric cancer) such as a normal or healthy cell or tissue or body fluid (to highlight its positivity) or a biological sample from the same subject and the same body fluid taken at a reference time such as diagnosis of the illness or earlier cycle(s) of treatment (to highlight its potential evolution).

In a preferred embodiment, the reference value according to the present invention is obtained in a sample from a healthy subject or in a sample from a subject suffering from gastric cancer previous to a treatment or performed in an earlier time of the treatment.

An "hypermethylation" is determined for example if the methylation value (amount) or status (level) of one of the biomarkers of the invention is significantly higher in the biological sample of the tested subject as compared with the methylation value (amount) or status (level) of the corresponding biomarker measured in a reference sample. A significantly higher amount or level of methylation of at least one of the biomarkers of the invention in the biological sample of a subject as compared with the normal amount or level of methylation in the reference sample is an indication that the tested subject has a gastric cancer, has a high risk to have gastric cancer or does not respond to a treatment.

A "significantly higher amount or level of methylation" refers to a methylation amount or level that is greater than the sum of the average and standard error of the assay employed to assess said amount or level.

In a preferred embodiment, said methylation amount is determined by Next Generation Sequencing (NGS) or by qPCR, preferably by dPCR.

As used herein, the term "dPCR" stands for "digital PCR". It is a refinement of conventional PCR methods, wherein the sample is separated into a large number of partitions and the PCR reaction is carried out in each partition individually. This separation allows a more reliable collection and sensitive measurement of nucleic acid amounts. The method has been demonstrated as useful for studying variations in gene sequences - such as copy number variants and point mutations - and it is routinely used for clonal amplification of samples for next-generation sequencing. More precisely, the PCR solution is divided into smaller reactions through a water oil emulsion technique, which are then made to run PCR individually. For example, the PCR sample can be partitioned into pico to nanoliter-size samples and encapsulated into oil droplets. The oil droplets are made using a droplet generator that applies a vacuum to each of the wells. Depending of the system used, from 5-10 million picoliter droplets (25-50 µL sample) to 20,000 oil nanoliter droplets (20 µL sample) can be created. Other type of compartments could also be used (such as microfabricated ones) as well as single tube limited dilutions.

In a more preferred embodiment, the level or amount of methylation of the *MSC-AS1* gene is determined by ddPCR by using the primers of SEQ ID NO:7-10, for example by using the primer pairs of SEQ ID NO:7-8 or of SEQ ID NO:9-10.

In a more preferred embodiment, the level or amount of methylation of the *ZNF790-AS1* gene is determined by ddPCR by using the primers of SEQ ID NO:11-14, for example by using the primer pairs of SEQ ID NO:11-12 or of SEQ ID NO:13-14.

In a more preferred embodiment, the level or amount of methylation of the *KCNA3* gene is determined by ddPCR by using the primer pair of SEQ ID NO:15-16.

In one exemplary embodiment, the present method can use methods for detecting gene expression (e.g., dPCR) that use fluorogenic probes to improve the specificity and/or the sensitivity of the detection of the PCR products that accumulate during PCR. Such assays are for example TaqMan^{®} gene expression assays using probes containing minor groove binding (MGB) moiety that enhances the Tₘ differential between matched and mismatched probes. In addition, these MGB probes may contain a non-fluorescent quencher (NFQ) that enhances spectral resolution when using multiple dyes in a reaction.

The probes that can be used in this preferred embodiment are reflected on Table 2 below and in SEQ ID NO:17-21.

In a more preferred embodiment, the level or amount of methylation of the *MSC-AS1* gene is determined by ddPCR by using the primers of SEQ ID NO:7-10 and the probes of SEQ ID NO:17-18.

In a more preferred embodiment, the level or amount of methylation of the *ZNF790-AS1* gene is determined by ddPCR by using the primers of SEQ ID NO:11-14 and the probes of SEQ ID NO:19-20.

In a more preferred embodiment, the level or amount of methylation of the *KCNA3* gene is determined by ddPCR by using the primer pair of SEQ ID NO:15-16 and the probes of SEQ ID NO:21.

Preferred combinations of biomarkers according to the invention are:
- *ZNF790-AS1* gene and *MSC-AS1* gene,
- *ZNF790-AS1* gene and *KCNA3* gene,
- *MSC-AS1* gene and *KCNA3* gene,
- *ZNF790-AS1* gene and *MSC-AS1* gene and *KCNA3* gene.

The method of the invention also comprises the step of comparing the level or amount of methylation of said gene(s) with the methylation level or amount of the same gene(s) that is determined in a reference sample as defined above.

If the *ZNF790-AS1* gene and the *MSC-AS1* gene are significantly hypermethylated in the biological sample of the tested subject as compared to the same biomarkers in the reference sample, then the tested subject has a gastric cancer or has a high risk to have or to develop a gastric cancer.

If the *ZNF790-AS1* gene and the *KCNA3* gene are significantly hypermethylated in the biological sample of the tested subject as compared to the same biomarkers in the reference sample, then the tested subject has a gastric cancer or has a high risk to have or to develop a gastric cancer.

If the *MSC-AS1* gene and the *KCNA3* gene are significantly hypermethylated in the biological sample of the tested subject as compared to the same biomarkers in the reference sample, then the tested subject has a gastric cancer or has a high risk to have or to develop a gastric cancer.

If the *ZNF790-AS1* gene, the *MSC-AS1* gene and the *KCNA3* gene are significantly hypermethylated in the biological sample of the tested subject as compared to the same biomarkers in the reference sample, then the tested subject has a gastric cancer or has a high risk to have or to develop a gastric cancer.

In another aspect, the biomarkers of the invention can be used to predict the outcome of gastric cancer patients. Also, they can be used to aid the skilled oncologist in the selection of appropriate treatments for maximizing the survival of the patients. Appropriate treatments are for example chemotherapeutic treatments, immunotherapeutic treatments, radiotherapeutic treatments and/or surgery. Preferably, the signature of the invention is generated before initiating a treatment.

Specifically, said patients have been treated or will be treated with chemotherapeutic drugs.

Said "chemotherapeutic drug" is typically an agent selected for example from an alkylating agent, an antimetabolite, a topoisomerase inhibitor, a platin based component, a specific kinase inhibitor, a hormone, a cytokine, an antiangiogenic agent, an antibody, an immunotherapy or a vascular disrupting agent.

The present invention also relates to a method for predicting the clinical outcome of a subject afflicted with gastric cancer, said method comprising:
a) determining the level or amount of methylation of at least one gene selected from the group consisting of *ZNF790-AS1* gene, *MSC-AS1* gene, and *KCNA3* gene, in a biological sample of said subject, and comparing same to a reference value,
b) predicting the clinical outcome based on the comparison of step a).

If the *MSC-AS1* gene, the *KCNA3* gene and/or *ZNF790-AS1* gene or a combination of these genes is / are significantly hypermethylated in the biological sample of the tested subject as compared to the same biomarker(s) in the reference sample, then the tested subject is likely to have a bad clinical outcome (short survival, metastasis, etc.).

Conversely, if the *MSC-AS1* gene, the *KCNA3* gene and/or *ZNF790-AS1* gene or a combination of these genes is / are not significantly hypermethylated or similar or less hypermethylated in the biological sample of the tested subject as compared to the same biomarker(s) in the reference sample, then the tested subject is likely to have a good clinical outcome.

The present invention also relates to a method for adapting the therapeutic regimen for a subject suffering from gastric cancer, said method comprising:
a) determining the level or amount of methylation of at least one gene selected from the group consisting of the *ZNF790-AS1* gene, the *MSC-AS1 gene,* and the *KCNA3* gene, in a biological sample of said subject, and
b) comparing same to a level or amount of methylation of the same biomarker as selected in step a), in a biological sample of said subject after surgery or after a treatment has been administrated to the subject,
c) adapting/modifying the therapeutic regimen of said subject based either on a) only or on the comparison of step a) and b).

In particular, said surgery or therapeutic regimen is acknowledged to be efficient if the level or amount of said biomarker(s) is significantly inferior or equal to the level or amount of said biomarker(s) determined before said treatment.

By contrast, said therapeutic regimen should be changed if the level or amount of said biomarker(s) has increased under treatment as compared with the level or amount of said biomarker(s) determined before said treatment (or determined in an earlier time of treatment).

This aspect of treatment strategy is a crucial goal in a context of personalized medicine in order to improve survival while maintaining the quality of life and avoiding needless toxic effects of an ineffective treatment.

The present invention also relates to a method for monitoring the progress of gastric cancer in a subject that has been diagnosed for gastric cancer, said method comprising:
a) determining the level or amount of methylation of at least one gene selected from the group consisting of the *ZNF790-AS1* gene, the *MSC-AS1* gene, and the *KCNA3* gene, in a biological sample of said subject, at a first time point,
b) determining the level or amount of methylation of said gene selected previously in the step a), in a biological sample of said subject, at a second time point, and
c) comparing the level or amount of methylation determined in step a) to the level or amount determined in step b).

It can be concluded that the malignancy of the gastric cancer is worsening if the level or amount of methylation determined in step b) is significantly higher than the level or amount determined in step a). In other words, the tested subject has a disease that evolves badly, even though he / she may be treated already.

If the subject is treated already, a first sample may have been taken from the subject prior to treatment for gastric cancer and a second sample may have been taken from the subject after being treated for gastric cancer. Consequently, said first time point is preferably prior to treatment of said subject and said second time point is after said subject has been treated for gastric cancer.

In this case, the method of the invention can be used for assessing the efficiency of said treatment.

Response to a treatment is more preferably defined according to RECIST 1.1 criteria [Mandard et al, 1994]. A Complete Response (CR) is defined as a disappearance of all target lesions. Any pathological lymph nodes (whether target or non-target) must have reduction in short axis to<10 mm. A Partial Response (PR) is defined as at least a 30% decrease in the sum of diameters of target lesions, taking as reference the baseline sum diameters. A Progressive Disease (PD) is defined as at least a 20% increase in the sum of diameters of target lesions, taking as reference the smallest sum on study (this includes the baseline sum if that is the smallest on study). In addition to the relative increase of 20%, the sum must also demonstrate an absolute increase of at least 5 mm (Note: the appearance of one or more new lesions is also considered progression). A Stable Disease (SD) is defined as neither sufficient shrinkage to qualify for PR nor sufficient increase to qualify for PD, taking as reference the smallest sum diameters while on study. Time of the evaluation often depends of the disease (it is usually comprised between 6 weeks and 3 months for gastric cancer).

In the context of the invention, if the methylation of the *MSC-AS1* gene, the *KCNA3* gene and/or *ZNF790-AS1* gene or a combination of these genes is / are significantly decreased in the biological sample acquired after the treatment has been administered as compared to the same biomarker(s) in a sample acquired before the treatment has been administered, then the tested subject is likely to be responding efficiently to the tested treatment (therefore being a "responder" as defined in the "complete response" RECIST criteria described above).

Conversely, if the methylation of the *MSC-AS1* gene, the *KCNA3* gene and/or *ZNF790-AS1* gene or a combination of these genes is / are significantly increased in the biological sample acquired after the treatment has been administered, as compared to the same biomarker(s) in a sample acquired before the treatment has been administered, then the tested subject is likely not to be responding efficiently to the tested treatment (therefore being a "non-responder").

As used herein, a "non-responder" is considered as a patient with a progressive disease or a stable disease as defined according to RECIST 1.1 criteria.

As used herein, "treatments" may include some combination of surgery, chemotherapy, radiation therapy and targeted therapy. In particular, multidisciplinary meeting should be planned before any decision of treatment. The pretherapeutic assessment should include physical examination with performance status, nutritional evaluation, cardiac function (if cardiotoxic chemotherapy is planned), pulmonary function (if surgery with thoracotomy is envisaged) and renal function. The treatment depends on the tumor stage. For example, for localized tumor, treatment is based on surgery in combination with perioperative chemotherapy or postoperative chemoradiotherapy. In metastatic disease, treatment is based on palliative chemotherapy combining fluoropyrimidine (capecitabine or 5-fluorouracile) and platinum salts (cisplatine or oxaliplatine). The trastuzumab, a monoclonal antibody blocking the HER2 receptor, should be used in combination with palliative chemotherapy for patients with HER2 positive tumor determined by immunohistochemistry or FISH.

In all the methods of the invention, the biomarkers of the invention, their combination with other genes, the tested subjects, the biological sample, and all the other parameters and definitions are those defined above.

### Kits of the invention

The present invention furthermore provides diagnostic kits and tools for determining the hypermethylation biomarkers of the invention in order to diagnose gastric cancer.

First, the present invention relates to nucleic acids which are useful to detect the hypermethylation regions highlighted above. More particularly, the invention pertains to isolated nucleic acids having a sequence of at least 10, 12, 15, 20, 30, 40 or 50 consecutive nucleotides (e.g. 10 to 30 nucleotides or 10 to 25 nucleotides) of the sequence SEQ ID NO:1 or SEQ ID NO:2 or SEQ ID NO:3 or SEQ ID NO:4 or SEQ ID NO:5 or SEQ ID NO:6.

Within the scope of the present invention, by "nucleic acid" is meant mRNA, genomic DNA or cDNA derived from mRNA.

These nucleic acids are preferentially primers or probes.

In another aspect, the present invention relates to a kit comprising said nucleic acids, preferably primers, for determining the level or amount of methylation in the nucleotide region of SEQ ID NO:1 or SEQ ID NO:4 in the *MSC-AS1* gene or in the nucleotide region of SEQ ID NO:2 or SEQ ID NO:5 in the *ZNF790-AS1* gene or in the nucleotide region of SEQ ID NO:3 or SEQ ID NO:6 in the *KCNA3* gene.

In a preferred embodiment, the present invention relates to a kit comprising said nucleic acids, preferably primers and/or probes, for determining the level or amount of methylation in the nucleotide region of SEQ ID NO:1 or SEQ ID NO:4 in the *MSC-AS1* gene or in the nucleotide region of SEQ ID NO:2 or SEQ ID NO:5 in the *ZNF790-AS1* gene or in the nucleotide region of SEQ ID NO:3 or SEQ ID NO:6 in the *KCNA3* gene.

As used herein, the term "primers" designates isolated nucleic acid molecules that can specifically hybridize or anneal to 5' or 3' regions of a target genomic region (plus and minus strands, respectively, or vice-versa). In general, they are from about 10 to 30 nucleotides in length and anneal at both extremities of a region containing about 50 to 200 nucleotides in length. Under appropriate conditions and with appropriate reagents, such primers permit the amplification of a nucleic acid molecule comprising the nucleotide sequence flanked by the primers. As they have to be used by pairs, they are often referred to as "primers pair" or "primers set" (cf. the pairs SEQ ID NO:7-8; SEQ ID NO:9-10, SEQ ID NO:11-12; SEQ ID NO:13-14; and SEQ ID NO:15-16).

As used herein, the term "probes" designates molecules that are capable of specifically hybridizing a genomic region of interest (e.g., of SEQ ID NO:1 or SEQ ID NO:2 or SEQ ID NO:3 or SEQ ID NO:4 or SEQ ID NO:5 or SEQ ID NO:6). They are useful to highlight the presence of said genomic region in biological samples. These probes may comprise at least one non-natural nucleotide, e.g., a peptide nucleic acid (PNA), a peptide nucleic acid having a phosphate group (PHONA), a bridged nucleic acid or locked nucleic acid (BNA or LNA), and a morpholino nucleic acid. Non-natural nucleotides also include chemically modified nucleic acids or nucleic acid analogs such as methylphosphonate-type DNA or RNA, phosphorothioate-type DNA or RNA, phosphoramidate-type DNA or RNA, and 2'-O-methyl-type DNA or RNA.

In a preferred embodiment, the probes of the invention comprise at least 15, consecutive nucleotides which are complementary of bisulfited SEQ ID NO:1 - SEQ ID NO:6 or fragments thereof. In a more preferred embodiment, the molecules which can be used as a probe according to the present invention have a total minimum size of 15 nucleotides. In an even more preferred embodiment, these molecules comprise between 15 and 30 nucleotides (in total).

For certain uses, the probes and primers of the invention may be labeled - directly or indirectly - with a detectable label. Said label may be of any kind, depending on the experiment which is to be performed. Said label may be a radioactive isotope (such as ³²P, ³³P, ³⁵S, ³H or ¹²⁵I, or a nonradioactive entity which is selected from ligands (such as biotin, avidin or streptavidin), dioxygenin, haptens, colorants and luminescent agents (such as radioluminescent, chemiluminescent, bioluminescent, fluorescent or phosphorescent agents). Preferably, 6-carboxyfluorescein (FAM), VIC, HEX and tetramethylrhodamine (TAMRA) are used. Non-labeled polynucleotide sequences may also be used, directly, as a probe or primer, for example in PCR-based processes (e.g., in quantitative PCR).

"Specific hybridization" is observed when a define molecule does not hybridize with any other genomic region than its target genomic region. Preferably, it hybridizes with its target region in high stringency conditions, i.e., when the temperature and ionic strength conditions are chosen so as to allow the hybridization between two complementary DNA fragments. By way of illustration, high stringency conditions can be as follows. The DNA-DNA or DNA-RNA hybridization is carried out in two steps: (1) prehybridization at 42°C for 3 hours in phosphate buffer (20 mM, pH 7.5) containing 5*SSC (1*SSC corresponds to a 0.15 M NaCl+0.015 M sodium citrate solution), 50% of formamide, 7% of sodium dodecyl sulfate (SDS), 10*Denhardt's, 5% of dextran sulfate and 1% of salmon sperm DNA; (2) actual hybridization for 20 hours at a temperature dependent on the size of the probe (i.e. 42°C. for a probe of size>100 nucleotides), followed by two 20-minute washes at 20°C. in 2*SSC+2% SDS and one 20-minute wash at 20°C. in 0.1*SSC+0.1% SDS. The final wash is carried out in 0.1*SSC+0.1% SDS for 30 minutes at 60°C for a probe of size>100 nucleotides. The high stringency hybridization conditions described above for a polynucleotide of defined size will be adjusted by those skilled in the art for oligonucleotides of greater or smaller size.

As used herein, the term "kit" refers to any system for delivering materials. In the context of the invention, it includes systems that allow the storage, transport, or delivery of reaction reagents (e.g., oligonucleotides, enzymes, etc. in the appropriate containers) and/or supporting materials (e.g., buffers, written instructions for performing the assay etc.) from one location to another. For example, kits include one or more enclosures (e.g., boxes) containing the relevant reaction reagents and/or supporting materials. The present kit can also include one or more reagents, buffers, hybridization media, nucleic acids, primers, nucleotides, probes, molecular weight markers, enzymes, solid supports, databases, computer programs for calculating dispensation orders and/or disposable lab equipment, such as multi-well plates, in order to readily facilitate implementation of the present methods. Enzymes that can be included in the present kits include nucleotide polymerases and the like. Solid supports can include beads and the like whereas molecular weight markers can include conjugatable markers, for example biotin and streptavidin or the like.

The kit of the invention more preferably contains primers targeting specifically the nucleotide region of SEQ ID NO:1 or SEQ ID NO:4 in the *MSC-AS1* gene or the nucleotide region of SEQ ID NO:2 or SEQ ID NO:5 in the *ZNF790-AS1* gene or the nucleotide region of SEQ ID NO:3 or SEQ ID NO:6 in the *KCNA3* gene.

In a preferred embodiment, the kit of the invention more preferably contains primers and probes targeting specifically the nucleotide region of SEQ ID NO:1 or SEQ ID NO:4 in the *MSC-*AS1 gene or the nucleotide region of SEQ ID NO:2 or SEQ ID NO:5 in the *ZNF790-AS1* gene or the nucleotide region of SEQ ID NO:3 or SEQ ID NO:6 in the *KCNA3* gene.

Said kit contains preferably the primers of SEQ ID NO:7-10, SEQ ID NO:11-14 or SEQ ID NO:15-16 for determining the level of amount of methylation in SEQ ID NO:1, SEQ ID NO:2 or SEQ ID NO:3 respectively or SEQ ID NO:4, SEQ ID NO:5 or SEQ ID NO:6 respectively and the probes of SEQ ID NO:17-18, SEQ ID NO:19-20 or SEQ ID NO:21 for determining the level of amount of methylation in SEQ ID NO:1, SEQ ID NO:2 or SEQ ID NO:3 respectively or SEQ ID NO:4, SEQ ID NO:5 or SEQ ID NO:6 respectively. More preferably, said kit contains the primer pairs SEQ ID NO:7-8 or SEQ ID NO:9-10 and probes SEQ ID NO:17-18 for determining the level or amount of methylation in SEQ ID NO:1 or SEQ ID NO:4, the primer pairs SEQ ID NO:11-12 or SEQ ID NO:13-14 and probes SEQ ID NO:19-20 for determining the level or amount of methylation in SEQ ID NO:2 or SEQ ID NO:5, or the primer pair SEQ ID NO:15-16 and probes SEQ ID NO:21 for determining the level or amount of methylation in SEQ ID NO:3 or SEQ ID NO:6.

The kit of the invention more preferably contains primers and probes for determining the level or amount of methylation in the following regions:
- in the nucleotide region of SEQ ID NO:1 or SEQ ID NO:4 in the *MSC-AS1* gene and in the nucleotide region of SEQ ID NO:2 or SEQ ID NO:5 in the *ZNF790-AS1* gene; or
- in the nucleotide region of SEQ ID NO:1 or SEQ ID NO:4 in the *MSC-AS1* gene and in the nucleotide region of SEQ ID NO:3 or SEQ ID NO:6 in the *KCNA3* gene; or
- in the nucleotide region of SEQ ID NO:3 or SEQ ID NO:6 in the *KCNA3* gene and in the nucleotide region of SEQ ID NO:2 or SEQ ID NO:5 in the *ZNF790-AS1* gene; or
- in the nucleotide region of SEQ ID NO:1 or SEQ ID NO:4 in the *MSC-AS1* gene and in the nucleotide region of SEQ ID NO:3 or SEQ ID NO:6 in the *KCNA3* gene and in the nucleotide region of SEQ ID NO:2 or SEQ ID NO:5 in the *ZNF790-AS1* gene.

Exemplary primers that can be used in the kit of the invention for determining hypermethylation of the nucleotide region of SEQ ID NO:1 or SEQ ID NO:4 in the *MSC-AS1* gene are highlighted on SEQ ID NO:7-10. Two exemplary probes that can also be used are provided in Table 2 and in SEQ ID NO:17-18.

Exemplary primers that can be used in the kit of the invention for determining hypermethylation of the nucleotide region of SEQ ID NO:2 or SEQ ID NO:5 in the *ZNF790-AS1* gene are highlighted on SEQ ID NO:11-14. Two exemplary probes that can also be used are provided in Table 2 and in SEQ ID NO:19-20.

Exemplary primers that can be used in the kit of the invention for determining hypermethylation of the nucleotide region of SEQ ID NO:3 or SEQ ID NO:6 in the *KCNA3* gene are highlighted on SEQ ID NO:15-16. One exemplary probe that can also be used is provided in Table 2 and in SEQ ID NO:21.

These exemplary primers and probes are summarized in Tables 1 and 2 below.

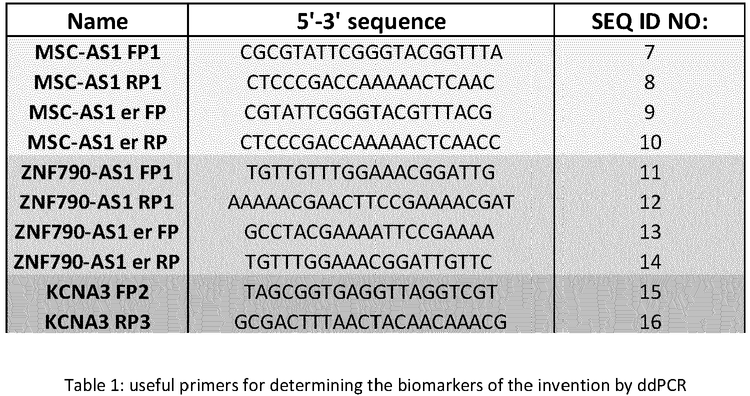

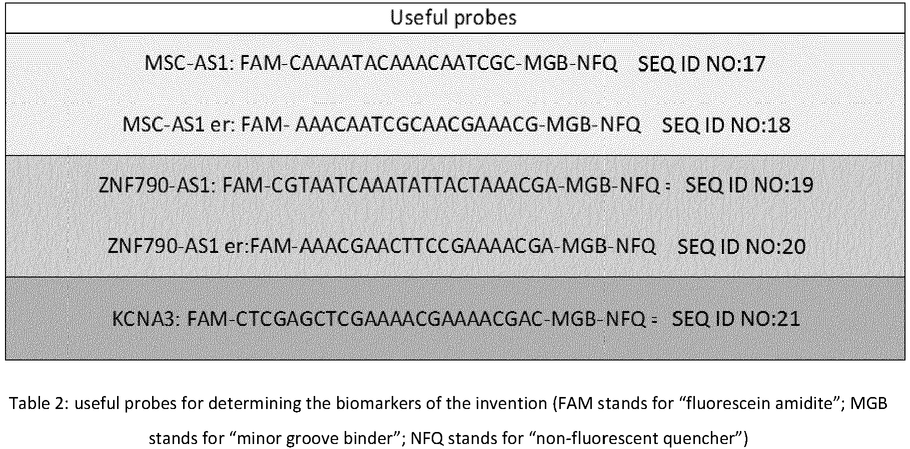

The present invention also relates to a microarray carrying nucleotides targeting specifically the nucleotide region of SEQ ID NO:1 or SEQ ID NO:4 in the *MSC-AS1* gene or the nucleotide region of SEQ ID NO:2 or SEQ ID NO:5 in the *ZNF790-AS1* gene or the nucleotide region of SEQ ID NO:3 or SEQ ID NO:6 in the *KCNA3* gene. In a preferred embodiment, said microarray contains any primers and/or probes of SEQ ID NO:7-21.

According to the invention, a "nucleic microarray" consists of different nucleic acid probes that are attached to a substrate, which can be a microchip, a glass slide or a microsphere-sized bead. A microchip may be constituted of polymers, plastics, resins, polysaccharides, silica or silica-based materials, carbon, metals, inorganic glasses, or nitrocellulose. Probes can be nucleic acids such as cDNAs ("cDNA microarray") or oligonucleotides ("oligonucleotide microarray"), and the oligonucleotides may be about 10 to about 40 base pairs or less in length. Typically, the exemplary primers and probes mentioned above can be attached to said substrate.

In a preferred embodiment, the nucleic acid microarray of the invention is an oligonucleotide microarray carrying oligonucleotides that can specifically hybridize with one, two, or three of the methylation regions of SEQ ID NO:1-6. In a more preferred embodiment, the nucleic acid microarray of the invention contains at least one primer SEQ ID NO:7-10 for determining the level or amount of methylation in SEQ ID NO:1 or SEQ ID NO:4, a primer of SEQ ID NO:11-14 for determining the level or amount of methylation in SEQ ID NO:2 or SEQ ID NO:5, or a primer of SEQ ID NO:15-16 for determining the level or amount of methylation in SEQ ID NO:3 or SEQ ID NO:6.

In a more preferred embodiment, the nucleic acid microarray of the invention also carries nucleic acids specific for additional genes and optionally one or more housekeeping gene(s), but preferably consists of a maximum of 500, 400, 300, 200 preferably 100, 90, 80, 70 more preferably 60, 50, 45, 40, 35, 30, 25, 20, 15, 10, or even less (for instance 9, 8, 7, 6, 5, 4, 3, 2 or 1) distinct nucleic acids.

To determine if the biomarker of the invention is methylated or not, the tested sample is labelled, contacted with the nucleic acid microarray of the invention in hybridization conditions, leading to the formation of complexes between target nucleic acids that are complementary to probe sequences attached to the microarray surface. The presence of labelled hybridized complexes on the nucleic acid microarray is then determined. Many variants of the microarray hybridization technology are available to the man skilled in the art.

Suitable microarray oligonucleotides specific for any gene of SEQ ID NO: 1 to 6 may be designed, based on the genomic sequence of each gene, using any method of microarray oligonucleotide design known in the art. In particular, any available software developed for the design of microarray oligonucleotides may be used, such as, for instance, the OligoArray software (available at http://berry.engin.umich.edu/oligoarray/), the GoArrays software (available at http://www.isima.fr/bioinfo/goarrays/), the Array Designer software (available at http://www.premierbiosoft.com/dnamicroarray/index.html), the Primer3 software (available at http://frodo.wi.mit.edu/primer3/primer3_code.html), or the Promide software (available at http://oligos.molgen.mpg.de/), MethPrimer (http://www.urogene.org/cgi-bin/methprimer/methprimer.cgi) .

The present invention also relates to the use of the kits described above, or of the microarrays described above, or of the primers described above, or of the probes described above, for:
- diagnosing or identifying gastric cancer in a subject,
- predicting the clinical outcome in a subject afflicted with gastric cancer,
- determining the therapeutic regimen of a subject with gastric cancer, and/or
- monitoring the progress of gastric cancer in a subject being diagnosed for gastric cancer,
in a biological sample of a subject, as proposed in the methods of the invention.

As used herein, the terms ***"in vitro"*** and ***"ex vivo"*** are equivalent and refer to studies or experiments that are conducted using biological components (e.g., cells or population of cells) that have been isolated from their usual host organisms (e.g., animals or humans).

### Figure legends

**Figure 1** discloses the DNA methylation level of the selected biomarkers, ZNF790-AS1, MSC-AS1, and *KCNA3* in tumor and non-tumor adjacent tissues from GC patients by Methyl-seq (n=10). Paired non-parametric Wilcoxon test was used for the analysis of the hypermethylation difference between normal and adjacent tissue DNA.
**Figure 2** discloses the validation of DNA methylation of selected biomarkers, ZNF790-AS1, MSC-AS1, and *KCNA3* by ddPCR (n=20). Paired non-parametric Wilcoxon test was used for the analysis of the hypermethylation difference between tumor and adjacent normal tissue DNA.
**Figure 3** discloses the validation of DNA methylation of *MSC-AS1* biomarker in GC patients by ddPCR. The difference of DNA methylation in GC patient plasma (n=22) and healthy individuals plasma (n=10) was tested by ddPCR. Mann-whitney test was used for the analysis of the significance.
**Figure 4** discloses the survival probability of patients according to the detected quantity of methylated DNA. The GC patients were separated into three tertiles depending on the quantity of copy of methylated sequences detected per milliliter of plasma. Log-rank, tft: logrank trend test.

### Examples

### Materials and Methods:

### Patients and healthy control individuals

Matched tumor and adjacent non-tumor tissue biopsies from 20 gastric cancer patients were included in this study. Plasma samples from 33 healthy individuals were purchased from Biological Specialty Corporation (Colmar, USA) and BioPredict.Inc (Oradell, USA). Plasma from 55 advanced gastric cancer patients were collected in EDTA tubes. This study was approved by the local ethics committee and informed written consent was obtained from all the patients.

### Tumor sample preparation, storage, DNA extraction, and quantification

Tumor and adjacent non-tumor tissue biopsies were flash frozen in liquid nitrogen immediately after resection until further analysis. Each tumor was reviewed by a pathologist and the tumor cell content was assessed by hematoxylin-eosin-safran staining. DNA was extracted with the QlAampDNAMini Kit (Qiagen) according to the manufacturer's instructions. DNA concentration was measured by Qubit 2.0 fluorometer (Invitrogen, Life Technologies) with the use of the dsDNA BR Assay (Invitrogen). Extracted DNA samples were stored at -20 °C before testing.

### Plasma sample preparation, storage, DNA extraction, and quantification

Plasma samples of healthy individuals were received in dry ice, aliquoted and immediately frozen at -80 °C. Before extraction, plasma samples were centrifuged at 3000g for 10 min and then extracted with the use of the QIAmp Circulating Nucleic Acid Kit (Qiagen) or the ccfDNA Plasma kit (Promega) by RSC Maxwell instrument according to the manufacturer's instructions. Plasma form gastric cancer patients were extracted with the use of the ccfDNA Plasma kit (Promega) by RSC Maxwell instrument or the QIAmp Circulating Nucleic Acid Kit (Qiagen). The quantity of DNA was measured by Qubit 2.0 fluorometer (Invitrogen, Life Technologies) with the use of the dsDNA HS Assay (Invitrogen). Extracted DNA samples were stored at -20 °C before testing.

### Buffy coat preparation, DNA extraction, and quantification

Buffy coat was prepared from whole blood of patients that do not suffer from gastric cancer or from healthy individuals and then extracted with the use of the QIAmp Circulating Nucleic Acid Kit (Qiagen) according to the manufacturer's instructions. The quantity of DNA was measured by Qubit 2.0 fluorometer (Invitrogen, Life Technologies) with the use of the dsDNA BR Assay (Invitrogen). Extracted DNA samples were stored at -20 °C before testing.

### Genome-wide DNA methylome profiling in gastric cancer patients by Methyl-seq

DNA methylome libraries were prepared with the use of SeqCap Epi CpGiant Enrichment kit (Roche) according to the manufacturer' instructions. Briefly, 500ng to 1µg of tumor or non-tumor tissue DNA was fragmented with the use of Bioruptor (Diagenode s.a, Belgium). NGS adaptors were ligated to the fragmented DNA followed by the bisulfite conversion treatment with the use of EZ DNA Methylation-Lightning Kit (Zymo research). After several steps of purification and amplification, bisulfite-converted samples were hybridized with SeqCap Epi probe pool and then captured by SeqCap Pure Capture Beads. Post capture amplification was applied before passing the samples to the NextseqTM 500 sequencer (Illumina).

### Analysis and identification of DNA methylation biomarkers based on Methyl-seq data

Following a quality control, sequence data was aligned to bisulfite converted reference genome and analyzed for differentially methylated CpG sites (DMC) and differentially methylated regions (DMR) between tumor and non-tumor DNA with the use of Methylene: an R script. DMRs are the regions containing at least 5 DMCs in more than 80% of the samples. A list of DMRs was generated including different information: the level of methylation in non-tumor tissue DNA, the level of methylation in tumor tissue DNA, the difference of methylation level between tumor and non-tumor tissue DNA, q value, gene symbol corresponding to the DMRs identified etc. Based on this list of DMR generated, different parameters were then used for identifying DNA methylation biomarkers for gastric cancer patients. First, all the DMRs with a q value less than 0.05 were selected. Second, DMRs with the difference of methylation level more than 40% between tumor and non-tumor DNA were chosen. Third, DMRs satisfied with the previous 2 criteria were then selected by the condition that the methylation level in non-tumor DNA was less than 5%. Following this, DMRs in the intergenic region were removed from the candidate biomarkers list and DMRs annotated with the genes were verified in the public database to remove those which have been described before.

### Tissue DNA, buffy coat DNA and Plasma ccfDNA bisulfite conversion

Tissue DNA, buffy coat DNA and plasma ccfDNA were modified by bisulfite using the EZ DNA Methylation-Gold Kit (Zymo Research). In brief, bisulfite reaction was carried out in a thermocycler at 98 °C for 12 min and 64 °C for 2 h35 min. The cleanup of bisulfite-converted DNA followed the recommendations of the manufacturer and converted DNA was eluted in M-Elution Buffer and stored at -20 °C.

### Detection of methylation changes of selected biomarkers by droplet-based digital PCR

The hypermethylation of selected biomarkers in tumor DNA was validated by ddPCR. Duplex format was used to analyze hypermethylation with albumin for normalizing the DNA amount. Primers and probes were listed in the Table 1 and 2 above. In particular, the primers pair of SEQ ID NO: 9-10 and probes of SEQ ID NO: 18 for MSC-AS1, primers pair of SEQ ID NO: 11-12 and probes of SEQ ID NO: 19 for ZNF790-AS1 and primers pair of SEQ ID NO: 15-16 and probes of SEQ ID NO: 21 for KCNA3 were used.

DNA methylation of targeted sequences was analyzed by ddPCR using either the Raindrop ddPCR system (Raindance technologies, today Bio-Rad) or the QX-200 platform (BIO-RAD Technologies). In brief, when using the first system, 12.5µL Kapa probe Fast qPCR master mix (Kapa Biosystems) was mixed with the assay solution containing: 0.75µL 40 mM dNTP Mix (New England BioLabs), 0.5µL 25 mM MgCl2, 1µL 25x Droplet Stabilizer (RainDance Technologies), 1.25µL 20x Assay Mix containing 8µM of forward and reverse primers, 4µM of 6-FAM and 12µM of VIC Taqman^{®} labeled-probes, and target modified DNA template to a final reaction volume of 25µL. When possible, a minimum of 10ng of modified DNA was used in each reaction.

When using the second system, the mixture of PCR reagents (BIO-RAD Technologies) was prepared following manufacturer's recommendations. A triplex panel was developed to detect the KCNA3 and MSC-AS1 methylated target sequences as well as the unmethylated Albumin sequence as a reference gene. A duplex assay detecting ZNF790-AS1 methylated target sequence as well as the unmethylated Albumin sequence as a reference gene was also developed. In the triplex panel, final concentrations in reaction mix of forward and reverse primers were 400nM for albumin and KCNA3 and 600nM for MSC-AS1. 600nM of VIC Taqman^{®} labeled-probes were used to detect albumin unmethylated sequences, 400nM of FAM Taqman^{®} labeled-probes were used to detect MSC-AS1 methylated sequences and 200 of both FAM and VIC Taqman^{®} labeled-probes were used to detect KCNA3 methylated sequences (see Method 1 for primer and probe sequences). The duplex detecting the target sequence of ZNF 790-AS1 was also optimized for the QX-200 platform. In this assay, final concentrations in reaction mix of forward and reverse primers were 400nM for albumin and 600nM for ZNF790-AS1. Moreover, 600nM of VIC Taqman^{®} labeled-probes were used to detect albumin unmethylated sequences, 400nM of FAM Taqman^{®} labeled-probes were used to detect ZNF790-AS1 methylated sequences. The PCR step for ddPCR was performed on a BIO-RAD C1000 or S1000 using the following program: 10 min at 95°C (using a 2.5°C/second ramp rate), followed by 45 cycles of: 94°C, 30 s and 57.3°C (triplex assay) or 58.4°C (duplex assay), 1 min (using a 2.5°C/second ramp rate), with an ultimate step of 10 min at 98°C. After completion, the emulsions were either stored at 4°C or processed immediately to measure the end-point fluorescence signal from each droplet. Data was analyzed using the Quantasoft BIO-RAD software as described by the manufacturer. Populations were clustered according to fluorescence levels, allowing to precisely count both tumor and normal amplifiable DNA molecules.

For the different assays, limit of blank (LOB) was calculated as described previously [Taly, V., et al. 2013] using commercial DNA extracted from whole Blood (Promega) for both duplex and triplex developed assays. It is defined by the frequency of positive droplets measured in normal control DNA samples with no hyper-methylated DNA present (n=10 to 23 for each assay depending on samples availability). The calculated LOB was subtracted from each sample for calculating their methylation level.

The sample analysis was performed following the procedure described earlier [Taly, V., et al. 2013]. Samples were considered positive when the number of observed droplets was higher than LOB value. The methylation level of each sample was calculated as the ratio of the number of droplets containing methylated sequences over the number of droplets containing albumin sequences.

Two DNA controls were used for ensuring the proper realization of the modification treatment (Positive control: universal hypermethylated DNA and negative control: normal human genomic DNA).

### Measurement of the methylation level of selected biomarkers in plasma circulating cell free DNA (ccfDNA) from healthy individuals and gastric cancer patients

DNA methylation of selected biomarkers in plasma from healthy individuals or gastric cancer patients was measured by ddPCR with the use of same reaction conditions as described before. Duplex format was used to analyze hypermethylation with albumin for normalizing the DNA amount. The same primers and probes were used as listed in the Table 1 and 2 above. In particular, the primers pair of SEQ ID NO: 9-10 and probes of SEQ ID NO: 18 for MSC-AS1, primers pair of SEQ ID NO: 11-12 and probes of SEQ ID NO: 19 for ZNF790-AS1 and primers pair of SEQ ID NO: 15-16 and probes of SEQ ID NO: 21 for KCNA3 were used.

### Calculation of detection sensitivity and specificity

The sum of the average and standard deviation of methylation level in non-tumor tissue DNA was used as the threshold for calculating sensitivity and specificity of each selected biomarker. Sensitivity is the percentage of the patients showing higher methylation level in tumor tissues than the threshold. Specificity is the percentage of the patients showing lower methylation level in non-tumor tissues than the threshold.

### Statistical analysis

Statistical analyses were performed using Prism Software (GraphPad Software Inc.) and R software 3.6.3 Studio. A P value ≤ 0.05 was considered as significant. For the analysis of the hypermethylation difference between normal and adjacent tissues, paired non-parametric Wilcoxon test was used. For the analysis of the hypermethylation difference between healthy and gastric cancer plasma ccfDNA, Mann-whitney test was used. The demographic characteristics of patients were compared by the Chi-square or Fisher's exact test. Continuous data were analyzed with the independent-samples t-test. Survival rates were calculated using the Kaplan-Meier method. Progression-free survival (PFS) was measured from the date of blood sampling used for ctDNA determination in the study to the first documented radiological progression (RECIST V1.1), or death, whichever occurred first. Patients lost to follow-up were censored at last follow-up visit. Association between outcomes and baseline characteristics was assessed with Cox proportional hazard models and hazard ratio (HR) with 95% CI were provided.

### Results

### Identification of DNA methylation biomarkers based on Methyl-seq data

A total of 20 samples (paired tumor and non-tumor tissue DNA) from 10 gastric cancer (GC) patients were analyzed for methylome profiles by Methyl-seq following SeqCap Epi CpGiant Enrichment libraries preparation. 187,166 DMR was identified in gastric tumor tissue DNA compared with non-tumor tissue DNA. Among them, 15,543 DMRs were with a q value less than 0.05. Following this, a more stringent threshold: difference of methylation level is more than 40% between tumor DNA and non-tumor DNA and the methylation level is less than 5% of methylation in non-tumor DNA was applied for the identification of potential DNA methylation biomarkers for gastric cancer. Finally, 3 potential biomarkers: *KCNA3, ZNF790-AS1, and MSC-*AS1 were identified.

### Hypermethylation of the selected biomarkers in tumor DNA from GC patients by Methyl-seq

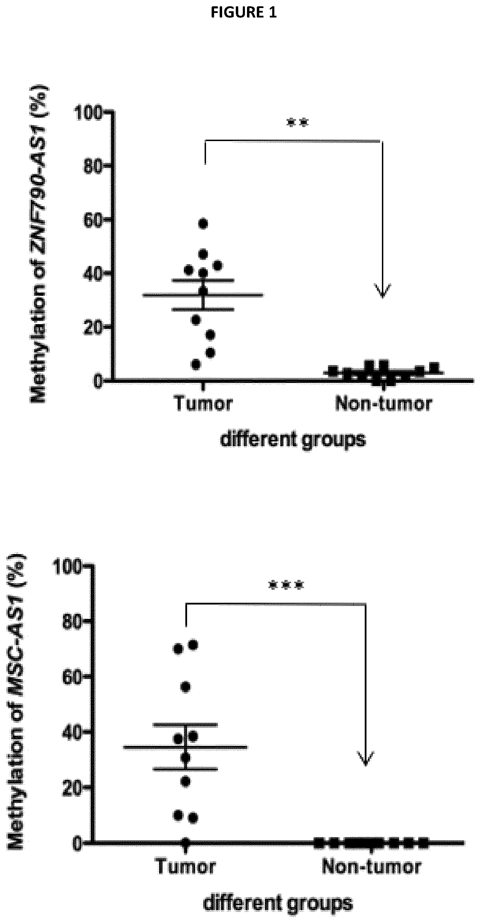

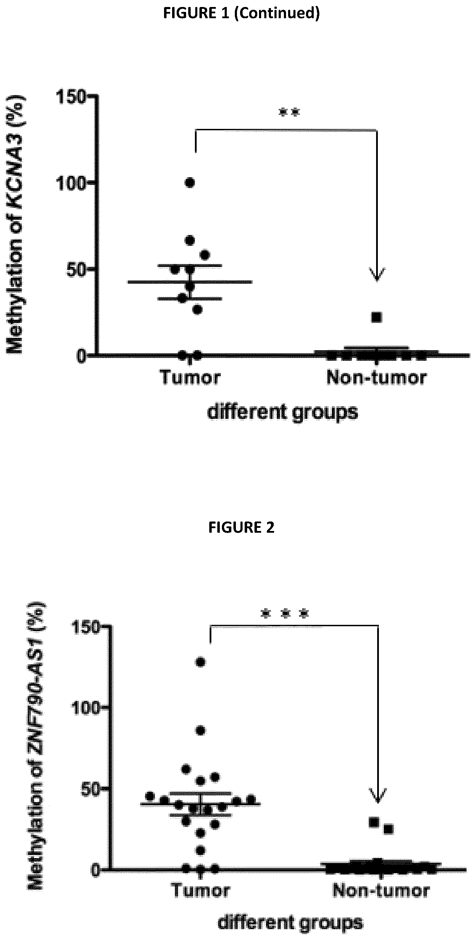

As shown in Figure 1, compared with those in non-tumor tissue DNA, methylation level of theselected biomarkers: *KCNA3, ZNF790-AS1, and MSC-AS1,* was significantly increased in gastric tumor tissue DNA. The detection sensitivity and specificity of all these biomarkers by Methyl-seq were more than 80%:

**Table 3: sensitivity and specificity of the biomarkers of the invention when assessed alone by MethylSeq**

| | **Methyl-seq** | | |
|---|---|---|---|
| | ***ZNF790-AS1*** | ***MSC-AS1*** | ***KCNA3*** |
| **Sensitivity** | **100** | **90** | **80** |
| **Specificity** | **80** | **100** | **90** |

### Validation of DNA methylation changes of selected biomarkers by ddPCR

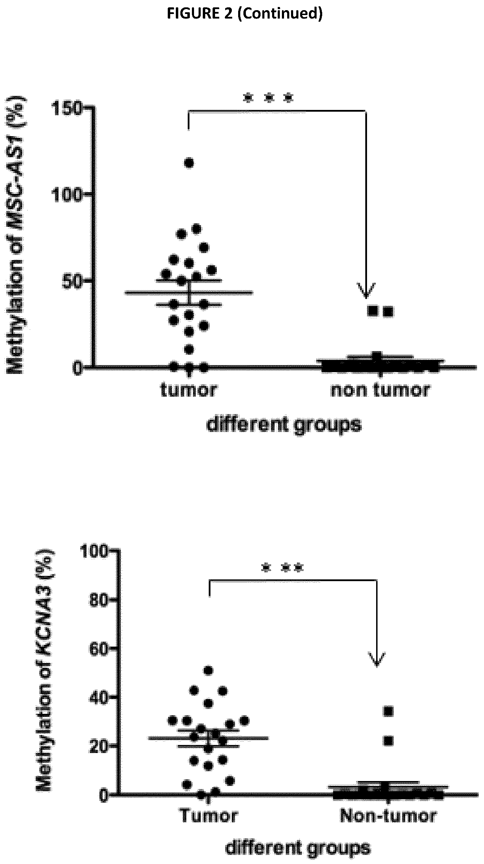

The difference of DNA methylation of several potential biomarkers in tumor and non-tumor tissue from GC patients was validated by ddPCR (n=20) with primers pair of SEQ ID NO: 9-10 and probes of SEQ ID NO: 18 for MSC-AS1, primers pair of SEQ ID NO: 11-12 and probes of SEQ ID NO: 19 for ZNF790-AS1 and primers pair of SEQ ID NO: 15-16 and probes of SEQ ID NO: 21 for KCNA3. Compared to those in non-tumor tissue, DNA methylation in tumor tissue was significantly increased as already demonstrated by Methyl-seq(Figure 2).

The detection sensitivity and specificity of the biomarkers *MSC-A1, KCNA3* and *ZNF-90-AS1* by ddPCR in gastric cancers were more than 80%:

**Table 4: sensitivity and specificity of the biomarkers of the invention when assessed alone by ddPCR**

| >Average (Non tumor) +1SD | KCNA3 (12,0%) | ZNF790-AS1 (11,82%) | MSC-AS1 (11,29%) |
|---|---|---|---|
| **sensitivity** | 80 | 85 | 85 |
| **Specificity** | 90 | 90 | 90 |

### Combination of different biomarkers to reach a higher sensitivity and specificity

The sensitivity and specificity of each individual biomarker by Methyl-seq and ddPCR are shown in Table 3 and Table 4 respectively. The highest sensitivity with the use of only one biomarker is 85%. To reach a higher detection sensitivity and specificity, different biomarkers can be combined (Table 5 and 6, results obtained by MethylSeq or ddPCR respectively). As targeted in the invention, the detection sensitivity by ddPCR can furthermore be improved to 100% by combining *MSC-AS1* with *ZNF790-AS1* or *MSC-AS1* with *KCNA3.*

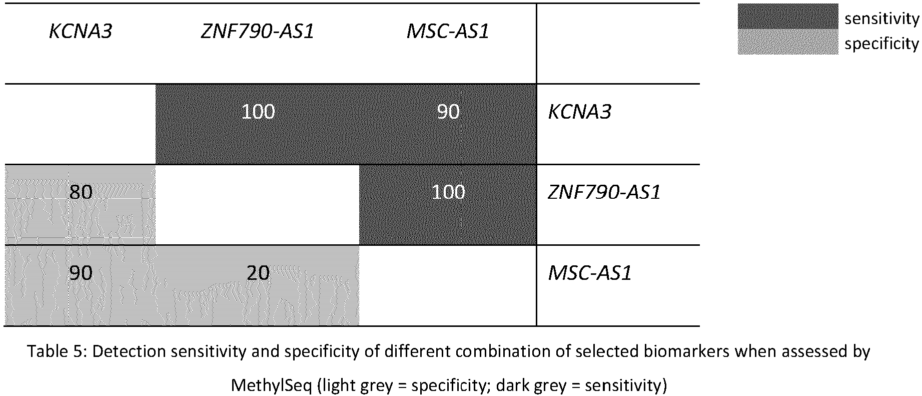

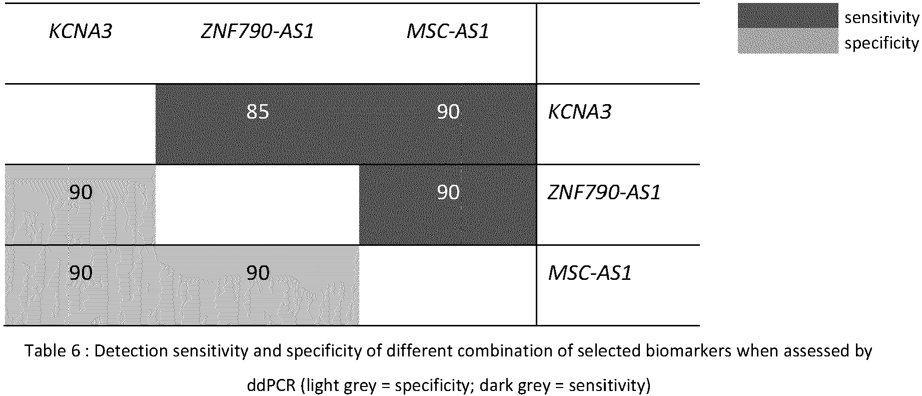

### Methylation profiles of the selected biomarkers in plasma ccfDNA and buffy coat fraction from healthy individuals and patients by ddPCR

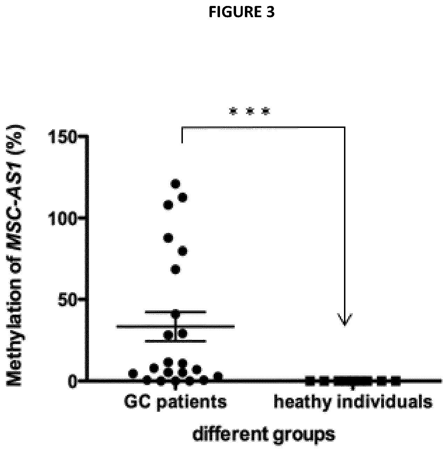

The purpose of these potential biomarkers is to detect DNA hypermethylation in gastric cancer patients but not in healthy individuals. In order to validate this, the methylation level in plasma ccfDNA from healthy individuals was investigated by ddPCR. No significant level of methylation was observed in healthy plasma ccfDNA for all these potential biomarkers: *KCNA3, ZNF790-AS1 and MSC-AS1* (Figure 3 for *MSC-AS1,* results for other markers (Table 7 and Table 9 for duplex and triplex assays respectively)). Moreover, DNA extracted from Buffy Coat (from patients that do not suffer from gastric cancer, from patients with gastric cancer or healthy individuals) was also tested with the markers and no methylation of the tested markers was observed in these samples (Table 8 and 10 for duplex and triplex assays respectively). The number of the samples ran for each biomarker depended on the availability of DNA. Buffy coat DNA 1-6 were from colorectal cancer patients, buffy coat DNA 7-16 were from healthy individuals and other buffy coats were from Gastric cancer patients. As mentioned above for LOB calculation, commercial DNA extracted from whole Blood (Promega) was also used to validate the assay. Added to buffy coat DNA, these controls were performed to ensure that the markers were not positive in cells contained in the blood ensuring no false positive in case of blood cell hemolysis (for example due to pre-analytical sample handling).

However, when tested on DNA extracted from plasma of healthy subjects and gastric cancer patients, methylation was observed only for gastric patients (see below) both using duplex and triplex developed assays leading to a potential specificity of 100% for the detection of GC in blood samples.

**Table 7. Methylation level of the selected biomarkers in healthy plasma circulating cell free (ccfDNA) measured by ddPCR in duplex assays. Methylation level of selected biomarkers was measured in healthy plasma ccfDNA (n=8 to 14 depending on the availability) by ddPCR.**

| **Sample ID** | **Observed droplets** | | **Methylation of *ZNF790-AS1* (%)** |
|---|---|---|---|
| | **Alb** | ***ZNF790-AS1*** | |
| Healthy plasma 1 | 136 | 0 | 0 |
| Healthy plasma 2 | 522 | 0 | 0 |
| Healthy plasma 3 | 416 | 0 | 0 |
| Healthy plasma 4 | 391 | 0 | 0 |
| Healthy plasma 5 | 810 | 0 | 0 |
| Healthy plasma 6 | 280 | 0 | 0 |
| Healthy plasma 7 | 327 | 0 | 0 |
| Healthy plasma 8 | 199 | 0 | 0 |

| **Sample ID** | **Observed droplets** | | **Methylation of *MSC-AS1* (%)** |
|---|---|---|---|
| | **Alb** | ***MSC-AS1*** | |
| Healthy plasma 9 | 319 | 0 | 0 |
| Healthy plasma 10 | 862 | 0 | 0 |
| Healthy plasma 11 | 136 | 0 | 0 |
| Healthy plasma 12 | 95 | 0 | 0 |
| Healthy plasma 13 | 141 | 0 | 0 |
| Healthy plasma 14 | 100 | 0 | 0 |
| Healthy plasma 15 | 140 | 0 | 0 |
| Healthy plasma 16 | 92 | 0 | 0 |
| Healthy plasma 17 | 349 | 0 | 0 |
| Healthy plasma 18 | 146 | 0 | 0 |

| **Sample ID** | **Observed droplets** | | **Methylation of *KCNA3* (%)** |
|---|---|---|---|
| | **Alb** | ***KCNA3*** | |
| Healthy plasma 19 | 124 | 0 | 0 |
| Healthy plasma 20 | 157 | 0 | 0 |
| Healthy plasma 21 | 377 | 0 | 0 |
| Healthy plasma 22 | 304 | 0 | 0 |
| Healthy plasma 23 | 271 | 0 | 0 |
| Healthy plasma 24 | 195 | 0 | 0 |
| Healthy plasma 25 | 155 | 0 | 0 |
| Healthy plasma 26 | 314 | 0 | 0 |
| Healthy plasma 27 | 123 | 0 | 0 |
| Healthy plasma 28 | 143 | 0 | 0 |
| Healthy plasma 29 | 273 | 0 | 0 |
| Healthy plasma 30 | 307 | 0 | 0 |
| Healthy plasma 31 | 273 | 0 | 0 |
| Healthy plasma 32 | 276 | 0 | 0 |

**Table 8. Methylation level of the selected biomarkers in healthy or CRC buffy coat DNA measured by ddPCR in duplex assays.**

| **Sample ID** | **Observed droplets** | | **Methylation of *ZNF790-AS1* (%)** |
|---|---|---|---|
| | **Alb** | ***ZNF790-AS1*** | |
| Buffy coat1 | 89308 | 0 | 0,00 |
| Buffy coat2 | 84986 | 0 | 0,00 |
| Buffy coat3 | 133999 | 0 | 0,00 |
| Buffy coat4 | 35100 | 0 | 0,00 |
| Buffy coat5 | 82174 | 0 | 0,00 |
| Buffy coat6 | 164001 | 0 | 0,00 |

| **Sample ID** | **Observed droplets** | | **Methylation of *MSC-AS1* (%)** |
|---|---|---|---|
| | **Alb** | ***MSC-AS1*** | |
| Buffy coat7 | 44320 | 1 | 0,002 |
| Buffy coat8 | 49069 | 0 | 0,00 |
| Buffy coat9 | 25621 | 0 | 0,00 |
| Buffy coat10 | 48497 | 0 | 0,00 |
| Buffy coat11 | 47815 | 0 | 0,00 |
| Buffy coat12 | 51857 | 3 | 0,006 |
| Buffy coat13 | 60317 | 0 | 0,00 |
| Buffy coat14 | 63723 | 0 | 0,00 |
| Buffy coat15 | 56384 | 0 | 0,00 |
| Buffy coat16 | 40207 | 0 | 0,00 |

| **Sample ID** | **Observed droplets** | | **Methylation of *KCNA3* (%)** |
|---|---|---|---|
| | **Alb** | ***KCNA3*** | |
| Buffy coat1 | 93139 | 0 | 0,00 |
| Buffy coat2 | 473631 | 0 | 0,00 |
| Buffy coat3 | 89761 | 0 | 0,00 |
| Buffy coat4 | 213596 | 0 | 0,00 |
| Buffy coat5 | 148803 | 0 | 0,00 |
| Buffy coat6 | 83607 | 0 | 0,00 |

**Table 9. Methylation level of the selected biomarkers in healthy plasma circulating cell free (ccfDNA) measured by ddPCR in triplex assays detecting target methylated sequences of MSC-AS1 and KCNA3 (n=8) by ddPCR.**

| **Sample ID** | **Observed droplets** | | **Methylation of MSC-AS1 (%)** | **Observed droplets** | **Methylation of *KCNA3* (%)** |
|---|---|---|---|---|---|
| | **Alb** | ***MSC-AS1*** | | ***KCNA3*** | |
| Healthy plasma 33 | 1512 | 0 | 0 | 0 | 0 |
| Healthy plasma 34 | 1650 | 0 | 0 | 0 | 0 |
| Healthy plasma 35 | 2054 | 0 | 0 | 0 | 0 |
| Healthy plasma 36 | 1486 | 0 | 0 | 0 | 0 |
| Healthy plasma 37 | 1798 | 0 | 0 | 0 | 0 |
| Healthy plasma 38 | 1672 | 0 | 0 | 0 | 0 |
| Healthy plasma 39 | 1313 | 0 | 0 | 0 | 0 |

**Table 10. Methylation level of the selected biomarkers in buffy coat DNA from GC patients measured by ddPCR in triplex assays detecting target methylated sequences of MSC-AS1 and KCNA3 (n=22).**

| **Sample ID** | **Observed droplets** | | **Methylation of MSC-AS1 (%)** | **Observed droplets** | **Methylation of *KCNA3* (%)** |
|---|---|---|---|---|---|
| | **Alb** | ***MSC-AS1*** | | ***KCNA3*** | |
| Buffy Coat 17 | 6327 | 0 | 0 | 0 | 0 |
| Buffy Coat 18 | 6341 | 0 | 0 | 0 | 0 |
| Buffy Coat 19 | 6616 | 0 | 0 | 0 | 0 |
| Buffy Coat 20 | 6149 | 0 | 0 | 0 | 0 |
| Buffy Coat 21 | 6977 | 0 | 0 | 0 | 0 |
| Buffy Coat 22 | 2752 | 0 | 0 | 0 | 0 |
| Buffy Coat 23 | 1908 | 0 | 0 | 0 | 0 |
| Buffy Coat 24 | 4093 | 0 | 0 | 0 | 0 |
| Buffy Coat 25 | 6274 | 0 | 0 | 0 | 0 |
| Buffy Coat 26 | 4375 | 0 | 0 | 0 | 0 |
| Buffy Coat 27 | 4332 | 0 | 0 | 0 | 0 |
| Buffy Coat 28 | 5749 | 0 | 0 | 0 | 0 |
| Buffy Coat 29 | 6740 | 3 | 0.04 | 0 | 0 |
| Buffy Coat 30 | 5498 | 0 | 0 | 2 | 0.03 |
| Buffy Coat 31 | 6726 | 0 | 0.02 | 0 | 0 |
| Buffy Coat 32 | 6117 | 0 | 0 | 0 | 0 |
| Buffy Coat 33 | 6771 | 0 | 0 | 0 | 0 |
| Buffy Coat 34 | 6701 | 1 | 0 | 0 | 0 |
| Buffy Coat 35 | 6283 | 0 | 0 | 2 | 0.03 |
| Buffy Coat 36 | 6618 | 0 | 0 | 0 | 0 |
| Buffy Coat 37 | 6821 | 0 | 0 | 0 | 0 |
| Buffy Coat 38 | 6746 | 0 | 0 | 0 | 0 |

### Monitoring progression of gastric cancer.

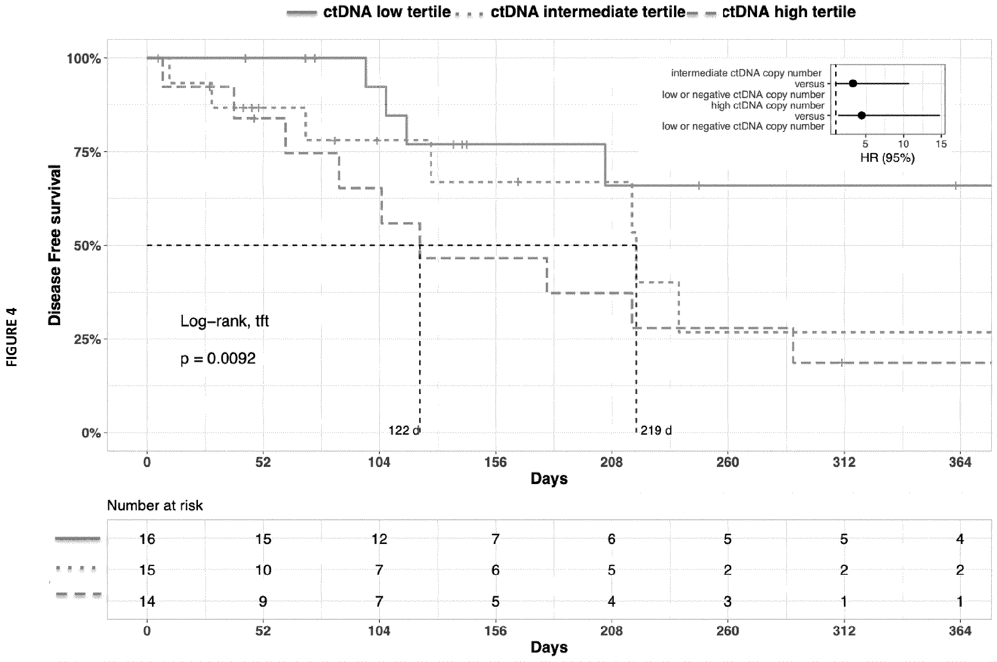

The methylation status of circulating cell free DNA as determined by the use of the markers MSC-A1 and KCNA3 demonstrated to be correlated with progression free survival (Figure 4). 55 patients in first, second or third line therapy were tested for the presence of methylated tumor DNA by the above described procedure. More than seventy-six percent (78%) were positive (n=43) for at least one marker. The methylation status of the GC patients was associated with higher progression free survival (see Figure 4). When dividing patients according to the quantity of detected methylated markers (tertiles, low, intermediate and high methylated ctDNA concentration in copy/mL of plasma), significant differences of disease free survival were observed. Indeed, increased probability of progression was observed for the group with highest concentration of methylated ctDNA vs the group with intermediate methylated ctDNA concentration vs the group with low methylated ctDNA concentration (Log-rank trend test, p=0.0092). Pertinence of these markers was further validated using univariate analysis. When analysis of methylation status (both when considering samples as positive vs negative for methylation or when considering the quantity of methylated ctDNA divided in three tertiles) gave significant results for prediction of cancer progression, analysis of potential GC markers (such as gender, Her2 amplification or tumor differenciation grade) did not give significant results. Table 11 summarizes the obtained data for patients treated in first and second line chemotherapy (n=48). Importantly quantity of ctDNA was also associated with progression of GC when taken as a continuous variable (p-value= 0.024).

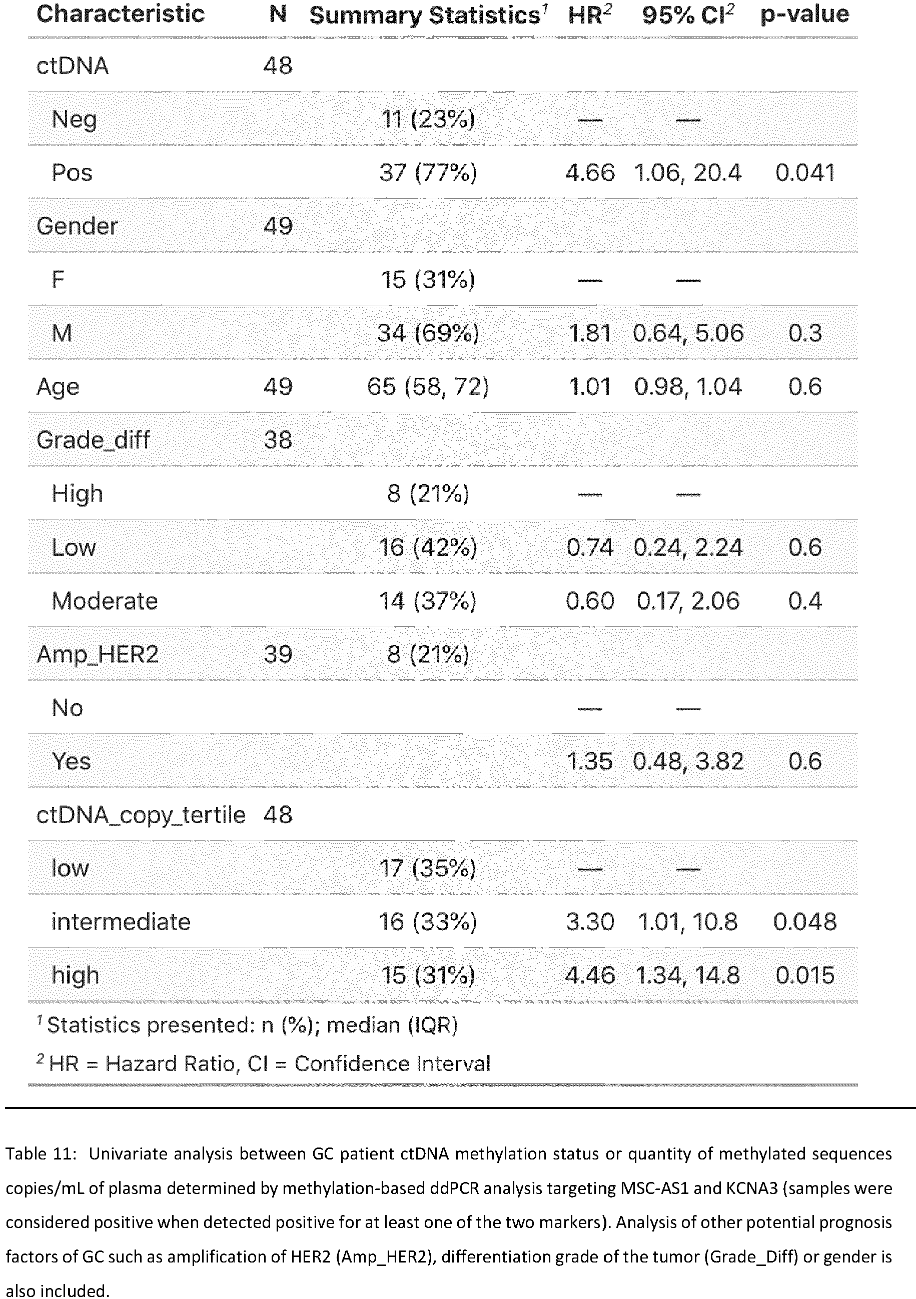

### Bibliographic references

Juhling, F., et al., metilene: fast and sensitive calling of differentially methylated regions from bisulfite sequencing data. Genome Res, 2016. 26(2): p. 256-62.
Garrigou, S., et al., A Study of Hypermethylated Circulating Tumor DNA as a Universal Colorectal Cancer Biomarker. Clin Chem, 2016. 62(8): p. 1129-39.
Milbury, C.A., et al., Determining lower limits of detection of digital PCR assays for cancer-related gene mutations. Biomol Detect Quantif, 2014. 1(1): p. 8-22.
Taly, V., et al., Multiplex picodroplet digital PCR to detect KRAS mutations in circulating DNA from the plasma of colorectal cancer patients. Clin Chem, 2013. 59(12): p. 1722-31.
Mikeska T, et al. DNA methylation biomarkers in cancer: progress towards clinical implementation. Expert Rev Mol Diagn. 2012 Jun; 12(5):473-87.
Jain, Wojdacz & Su, Challenges for the application of DNA methylation biomarkers in molecular diagnostic testing for cancer. Expert Rev Mol Diagn. 2013 Apr;13(3):283-94.
Delpu Y et al, DNA methylation and cancer diagnosis. Int J Mol Sci. 2013 Jul 18;14(7):15029-58
Mandard AM, Dalibard F, Mandard JC, et al. Pathologic assessment of tumor regression after preoperative chemoradiotherapy of esophageal carcinoma. Clinicopathologic correlations. Cancer. 1994;73(11):2680-268
Pinheiro LB et al, Evaluation of a Droplet Digital Polymerase Chain Reaction Format for DNA Copy Number Quantification. Anal. Chem. 2012, 84, 1003-1011

## Claims

1. An *in vitro* method for diagnosing or identifying gastric cancer in a subject, said method comprising determining the level or amount of methylation of the *MSC-AS1* gene and of at least one gene selected from the group consisting of : the *ZNF790-AS1* gene and the *KCNA3* gene, in a biological sample of said subject.

2. The method of claim 1, wherein if said genes are hypermethylated as compared with reference values, then said subject is diagnosed or identified as suffering from a gastric cancer.

3. The method of any of claims 1 to 2, wherein said biological sample is a blood sample of said subject.

4. The method of any of claims 1 to 3, wherein said methylation is determined by Next Generation Sequencing (NGS) or by qPCR, preferably by digital PCR (dPCR).

5. The method of any of claims 1 to 4, wherein said level or amount of methylation is determined in the nucleotide region of SEQ ID NO:4 in the *MSC-AS1* gene and in the nucleotide region of SEQ ID NO:5 in the *ZNF790-AS1* gene or in the nucleotide region of SEQ ID NO:6 in the *KCNA3* gene.

6. The method of any of claims 1 to 5, wherein said level or amount of methylation of the *MSC-AS1* gene and of the *ZNF790-AS1* gene or of the *KCNA3* gene is determined by dPCR by using the primers of SEQ ID NO:7-10, and SEQ ID NO:11-14 or SEQ ID NO:15-16 respectively, and using the probes of SEQ ID NO:17-18 and of SEQ ID NO:19-20 or of SEQ ID NO:21 respectively.

7. The method according to any of claims 1 to 6, comprising determining simultaneously or sequentially the level or amount of methylation of the *MSC-AS1* gene, the *ZNF790-AS1* gene and the *KCNA3* gene in a biological sample of said subject.

8. An *in vitro* method for monitoring the progress of gastric cancer in a subject being diagnosed for gastric cancer, said method comprising:
a) determining the level or amount of methylation of the *MSC-AS1* gene and of at least one gene selected from the group consisting of the *ZNF790-AS1* gene and the *KCNA3* gene, in a biological sample of said subject, at a first time point,
b) determining the level or amount of methylation of said genes selected previously in the step a), in a biological sample of said subject, at a second time point, and
c) comparing the level or amount of methylation determined in step a) to the level or amount determined in step b) or to reference values.

9. The method of claim 8, wherein the sample in step a) is obtained prior to the treatment for gastric cancer and the sample in step b) is obtained after said subject has been treated for gastric cancer.

10. The method according to any one of claims 8 to 9, wherein the level or amount of methylation is determined simultaneously or sequentially in the *MSC-AS1* gene, the *ZNF790-AS1* gene and the *KCNA3* gene in a biological sample of said subject.

11. The method of any one of claims 1 to 10, wherein said reference values are obtained in a healthy subject.

12. The method of any one of claims 9-11, for determining or adapting a therapeutic regimen suitable for a subject suffering from gastric cancer.

13. The method of claim 8, for predicting a clinical outcome of a subject afflicted with gastric cancer.

14. Use of a kit comprising primers targeting specifically :
i) the nucleotide region of SEQ ID NO:4 in the *MSC-AS1* gene and
ii) either the nucleotide region of SEQ ID NO:5 in the *ZNF790-AS1* gene or the nucleotide region of SEQ ID NO:6 in the *KCNA3* gene,
for diagnosing or identifying gastric cancer in a subject.

15. Use of the kit as defined in claim 14, for:
a) predicting a clinical outcome in a subject afflicted with gastric cancer,
b) determining the therapeutic regimen of a subject with gastric cancer, and/or
c) monitoring the progress of gastric cancer in a subject being diagnosed for gastric cancer.

## Patentansprüche

1. In-vitro-Verfahren zum Diagnostizieren oder Identifizieren von Magenkrebs bei einem Subjekt, wobei das Verfahren das Bestimmen des Niveaus oder der Menge der Methylierung des *MSC-AS1-*Gens und von mindestens einem Gen ausgewählt aus der Gruppe bestehend aus: dem *ZNF790-AS1*-Gen und dem *KCNA3-*Gen, in einer biologischen Probe dieses Subjekts umfasst.

2. Das Verfahren nach Anspruch 1, wobei, wenn die Gene im Vergleich zu Referenzwerten hypermethyliert sind, bei dem betreffenden Subjekt Magenkrebs diagnostiziert oder festgestellt wird.

3. Das Verfahren nach einem der Ansprüche 1 bis 2, wobei die biologische Probe eine Blutprobe des betreffenden Subjekts ist.

4. Das Verfahren nach einem der Ansprüche 1 bis 3, wobei diese Methylierung durch Next Generation Sequencing (NGS) oder durch qPCR, vorzugsweise durch digitale PCR (dPCR), bestimmt wird.

5. Das Verfahren nach einem der Ansprüche 1 bis 4, wobei dieses Niveau oder diese Menge der Methylierung in der Nukleotidregion von SEQ ID NO:4 im *MSC-AS1-*Gen und in der Nukleotidregion von SEQ ID NO:5 im *ZNF790-AS1*-Gen oder in der Nukleotidregion von SEQ ID NO:6 im *KCNA3-*Gen bestimmt wird.

6. Das Verfahren nach einem der Ansprüche 1 bis 5, wobei dieses Niveau oder diese Menge der Methylierung des *MSC-AS1*-Gens und des *ZNF790-AS1-*Gens oder des *KCNA3-*Gens durch dPCR unter Verwendung der Primer von SEQ ID NO:7-10 und SEQ ID NO:11-14 bzw. SEQ ID NO:15-16 und unter Verwendung der Sonden von SEQ ID NO:17-18 und von SEQ ID NO:19-20 bzw. von SEQ ID NO:21 bestimmt wird.

7. Das Verfahren nach einem der Ansprüche 1 bis 6, umfassend das gleichzeitige oder sequenzielle Bestimmen des Niveaus oder der Menge der Methylierung des MSC-AS1-Gens, des *ZNF790-AS1*-Gens und des *KCNA3-*Gens in einer biologischen Probe dieses Subjekts.

8. In-vitro-Verfahren zur Überwachung des Fortschreitens von Magenkrebs bei einem Subjekt, bei dem Magenkrebs diagnostiziert wurde, wobei dieses Verfahren umfasst:
a) Bestimmen des Niveaus oder der Menge der Methylierung des MSC-AS1-Gens und von mindestens einem Gen, das aus der Gruppe ausgewählt ist, die aus dem *ZNF790-AS1*-Gen und dem *KCNA3-*Gen besteht, in einer biologischen Probe des Subjekts zu einem ersten Zeitpunkt,
b) Bestimmen des Niveaus oder der Menge der Methylierung dieser zuvor in Schritt a) ausgewählten Gene in einer biologischen Probe dieses Subjekts zu einem zweiten Zeitpunkt, und
c)Vergleichen des in Schritt a) bestimmten Niveaus oder der Menge der Methylierung mit dem in Schritt b) bestimmten Niveau oder der Menge oder mit Referenzwerten

9. Das Verfahren nach Anspruch 8, wobei die Probe in Schritt a) vor der Behandlung von Magenkrebs gewonnen wird und die Probe in Schritt b) gewonnen wird, nachdem das Subjekt gegen Magenkrebs behandelt wurde.

10. Das Verfahren nach einem der Ansprüche 8 bis 9, wobei der Grad oder die Menge der Methylierung gleichzeitig oder sequentiell im *MSC-AS1-*Gen, im *ZNF790-AS1*-Gen und im *KCNA3*-Gen in einer biologischen Probe dieses Subjekts bestimmt wird.

11. Das Verfahren nach einem der Ansprüche 1 bis 10, wobei diese Referenzwerte bei einem gesunden Probanden ermittelt werden.

12. Das Verfahren nach einem der Ansprüche 9 bis 11 zum Bestimmen oder Anpassen eines für ein an Magenkrebs erkranktes Subjekt geeigneten Therapieplans.

13. Das Verfahren nach Anspruch 8 zum Vorhersagen eines klinischen Ergebnisses bei einem an Magenkrebs erkrankten Subjekt.

14. Verwendung eines Kits, das Primer umfasst, die speziell auf Folgendes abzielen:
a) die Nukleotidregion von SEQ ID NO:4 im *MSC-AS1-*Gen und
b) entweder die Nukleotidregion von SEQ ID NO:5 im *ZNF790-AS1*-Gen oder die Nukleotidregion von SEQ ID NO:6 im *KCNA3-Gen,*
zur Diagnose oder Identifizierung von Magenkrebs bei einem Subjekt.

15. Verwendung des Kits gemäß Anspruch 14 zum:
a) Vorhersagen eines klinischen Ergebnisses bei einem an Magenkrebs erkrankten Subjekt,
b) Bestimmen des Therapieplans eines Subjekts mit Magenkrebs und/oder
c) Überwachen des Fortschreitens von Magenkrebs bei einem Subjekt, bei dem Magenkrebs diagnostiziert wurde.

## Revendications

1. Méthode *in vitro* pour diagnostiquer ou identifier un cancer gastrique chez un sujet, ladite méthode comprenant la détermination du niveau ou de la quantité de méthylation du gène *MSC-AS1* et d'au moins un gène choisi dans le groupe constitué par le gène *ZNF790-AS1* et le gène *KCNA3,* dans un échantillon biologique dudit sujet.

2. Méthode selon la revendication 1, dans laquelle, si des gènes sont hyperméthylés en comparaison avec des valeurs de référence, alors ledit sujet est diagnostiqué ou identifié comme souffrant d'un cancer gastrique.

3. Méthode selon l'une quelconque des revendications 1 et 2, dans laquelle ledit échantillon biologique est un échantillon de sang dudit sujet.

4. Méthode selon l'une quelconque des revendications 1 à 3, dans laquelle ladite méthylation est déterminée par séquençage de nouvelle génération (NGS) ou par qPCR, de préférence par PCR digitale (dPCR).

5. Méthode selon l'une quelconque des revendications 1 à 4, dans laquelle ledit niveau ou quantité de méthylation est déterminé dans la région de nucléotides de la SEQ ID NO : 4 dans le gène *MSC-AS1* et dans la région de nucléotides de la SEQ ID NO : 5 dans le gène *ZFN790-AS1* ou dans la région de nucléotides de la SEQ ID NO : 6 dans le gène *KCNA3.*

6. Méthode selon l'une quelconque des revendications 1 à 5, dans laquelle ledit niveau ou quantité de méthylation du gène *MSC-AS1* et du gène *ZFN790-AS1* ou du gène *KCNA3* est déterminé par dPCR par utilisation des amorces des SEQ ID NO : 7 à 10 et des SEQ ID NO : 11 à 14 ou des SEQ ID NO : 15 et 16 respectivement, et par utilisation des sondes des SEQ ID NO : 17 et 18 et des SEQ ID NO : 19 et 20 ou de la SEQ ID NO : 21 respectivement.

7. Méthode selon l'une quelconque des revendications 1 à 6, comprenant la détermination simultanée ou successive du niveau ou de la quantité de méthylation du gène *MSC-AS1,* du gène *ZFN790-AS1* et du gène *KCNA3* dans un échantillon biologique dudit sujet.

8. Méthode *in vitro* pour surveiller l'évolution d'un cancer gastrique chez un sujet diagnostiqué avec un cancer gastrique, ladite méthode comprenant :
a) la détermination du niveau ou de la quantité de méthylation du gène *MSC-AS1* et d'au moins un gène choisi dans le groupe constitué par le gène *ZFN790-AS1* et le gène *KCNA3,* dans un échantillon biologique dudit sujet, à un premier point de temps,
b) la détermination du niveau ou de la quantité de méthylation desdits gènes sélectionnés antérieurement dans l'étape a), dans un échantillon biologique dudit sujet, à un deuxième point de temps, et
c) la comparaison du niveau ou de la quantité de méthylation déterminé dans l'étape a) au niveau ou à la quantité déterminé dans l'étape b) ou à des valeurs de référence.

9. Méthode selon la revendication 8, dans laquelle l'échantillon dans l'étape a) est obtenu avant le traitement pour un cancer gastrique et l'échantillon dans l'étape b) est obtenu après que ledit sujet a été traité pour un cancer gastrique.

10. Méthode selon l'une quelconque des revendications 8 et 9, dans laquelle le niveau ou la quantité de méthylation est déterminé simultanément ou successivement dans le gène *MSC-AS1,* le gène *ZFN790-AS1* et le gène *KCNA3* dans un échantillon biologique dudit sujet.

11. Méthode selon l'une quelconque des revendications 1 à 10, dans laquelle lesdites valeurs de référence sont obtenues chez un sujet en bonne santé.

12. Méthode selon l'une quelconque des revendications 9 à 11, pour déterminer ou adapter un régime thérapeutique convenant pour un sujet souffrant d'un cancer gastrique.

13. Méthode selon la revendication 8, pour prédire un résultat clinique chez un sujet affligé d'un cancer gastrique.

14. Utilisation d'une trousse comprenant des amorces ciblant spécifiquement :
i) la région de nucléotides de la SEQ ID NO : 4 dans le gène *MSC-AS1* et
ii) soit la région de nucléotides de la SEQ ID NO : 5 dans le gène *ZFN790-AS1* soit la région de nucléotides de la SEQ ID NO : 6 dans le gène *KCNA3,*
pour diagnostiquer ou identifier un cancer gastrique chez un sujet.

15. Utilisation de la trousse telle que définie dans la revendication 14, pour :
a) prédire un résultat clinique chez un sujet affligé d'un cancer gastrique,
b) déterminer le régime thérapeutique d'un sujet ayant un cancer gastrique, et/ou
c) surveiller l'évolution d'un cancer gastrique chez un sujet diagnostiqué avec un cancer gastrique.
